# EUROPEAN PATENT SPECIFICATION

(11) **EP 3 423 450 B1**
(45) Date of publication and mention of the grant of the patent: **27.11.2019**
(21) Application number: 17706829.3
(22) Date of filing: 28.02.2017
(51) Int. Cl.: C07D 413/04, C07D 213/85, C07D 413/14, A61K 31/5355, A61P 25/28, A61P 35/00, A61P 9/10, A61P 19/02, A61P 29/00, A61P 43/00

(54) **BACE 1 INHIBITORS**
BACE1-HEMMER
INHIBITEURS DE BACE1

(30) Priority: 01.03.2016 EP 16158075
(43) Date of publication of application: 09.01.2019
(73) Proprietor: F. Hoffmann-La Roche AG, 4070 Basel (CH)
(72) Inventor: HAAP, Wolfgang, 4070 Basel (CH); WOLTERING, Thomas, 4070 Basel (CH)
(74) Representative: Sauer, Frank
(86) International application number: PCT/EP2017/054541
(87) International publication number: WO 2017/148878

(56) References cited:
- WO-A1-2012/156284
- WO-A1-2013/027188
- WO-A1-2014/065434
- WO-A1-2014/114532
- WO-A1-2014/134341
- WO-A1-2014/166906
- WO-A1-2015/156421
- WO-A1-2016/001266

## Description

### Background Art

Alzheimer's disease (AD) is a neurodegenerative disorder of the central nervous system and the leading cause of a progressive dementia in the elderly population. Its clinical symptoms are impairment of memory, cognition, temporal and local orientation, judgment and reasoning but also severe emotional disturbances. There are currently no treatments available which can prevent the disease or its progression or stably reverse its clinical symptoms. AD has become a major health problem in all societies with high life expectancies and also a significant economic burden for their health systems.

AD is characterized by 2 major pathologies in the central nervous system (CNS), the occurrence of amyloid plaques and neurofibrillar tangles (Hardy et al., The amyloid hypothesis of Alzheimer's disease: progress and problems on the road to therapeutics, Science. 2002 Jul 19;297(5580):353-6*,* Selkoe, Cell biology of the amyloid beta-protein precursor and the mechanism of Alzheimer's disease, Annu Rev Cell Biol. 1994;10:373-403*).* Genetic ablation of the BACE1 gene in mice has clearly shown that its activity is essential for the processing of APP which leads to the generation of Aβ-peptides, in the absence of BACE1 no Aβ-peptides are produced. Both pathologies are also commonly observed in patients with Down's syndrome (trisomy 21), which also develop AD-like symptoms in early life. Neurofibrillar tangles are intracellular aggregates of the microtubule-associated protein tau (MAPT). Amyloid plaques occur in the extracellular space; their principal components are Aβ-peptides. The latter are a group of proteolytic fragments derived from the β-amyloid precursor protein (APP) by a series of proteolytic cleavage steps. Several forms of APP have been identified of which the most abundant are proteins of 695, 751 and 770 amino acids length. They all arise from a single gene through differential splicing. The Aβ-peptides are derived from the same domain of the APP but differ at their N- and C-termini, the main species are of 40 and 42 amino-acid length. There are several lines of evidence which strongly suggest that aggregated Aβ-peptides are the essential molecules in the pathogenesis of AD: 1) amyloid plaques formed of Aβ-peptides are invariably part of the AD pathology; 2) Aβ-peptides are toxic for neurons; 3) in Familial Alzheimer's Disease (FAD) the mutations in the disease genes APP, PSN1, PSN2 lead to increased levels of Aβ-peptides and early brain amyloidosis; 4) transgenic mice which express such FAD genes develop a pathology which bears many resemblances to the human disease. Aβ-peptides are produced from APP through the sequential action of 2 proteolytic enzymes termed β- and γ-secretase. β-Secretase cleaves first in the extracellular domain of APP approximately 28 amino acids outside of the trans-membrane domain (TM) to produce a C-terminal fragment of APP containing the TM- and the cytoplasmatic domain (CTFβ) CTFβ is the substrate for γ-secretase which cleaves at several adjacent positions within the TM to produce the Aβ peptides and the cytoplasmic fragment. The γ-secretase is a complex of at least 4 different proteins, its catalytic subunit is very likely a presenilin protein (PSEN1, PSEN2). The β-secretase (BACE1, Asp2; BACE stands for β-site APP-cleaving enzyme) is an aspartyl protease which is anchored into the membrane by a transmembrane domain (Vassar et al., Beta-secretase cleavage of Alzheimer's amyloid precursor protein by the transmembrane aspartic protease BACE, Science. 1999 Oct 22;286(5440):735). It is expressed in many tissues of the human organism but its level is especially high in the CNS. Genetic ablation of the BACE1 gene in mice has clearly shown that its activity is essential for the processing of APP which leads to the generation of Aβ-peptides, in the absence of BACE1 no Aβ-peptides are produced (Luo et al., Mice deficient in BACE1, the Alzheimer's beta-secretase, have normal phenotype and abolished beta-amyloid generation, Nat Neurosci. 2001 Mar;4(3):231-2*,* Roberds et al., BACE knockout mice are healthy despite lacking the primary beta-secretase activity in brain: implications for Alzheimer's disease therapeutics, Hum Mol Genet. 2001 Jun 1;10(12):1317-24). Mice which have been genetically engineered to express the human APP gene and which form extensive amyloid plaques and Alzheimer's disease like pathologies during aging fail to do so when β-secretase activity is reduced by genetic ablation of one of the BACE1 alleles (McConlogue et al., Partial reduction of BACE1 has dramatic effects on Alzheimer plaque and synaptic pathology in APP Transgenic Mice. J Biol Chem. 2007 Sep 7;282(36):26326). It is thus presumed that inhibitors of BACE1 activity can be useful agents for therapeutic intervention in Alzheimer's disease (AD).

The accumulation and aggregation of Aβ-peptides is one of the underlying causes of AD. APP is cleaved by BACE1 to generate a soluble N-terminal ectodomain of APP (sAPPβ) and a membrane-bound fragment C99. This C99 fragment is subsequently cleaved by γ-secretase to generate various Aβ-species, such as Aβ40 and Aβ42. These Aβ-species are the most neurotoxic species and a responsible to form the amyloid plaques.

Furthermore, the formation, or formation and deposition, of β-amyloid peptides in, on or around neurological tissue (e.g., the brain) are inhibited by the present compounds, i.e. inhibition of the Aβ-production from APP or an APP fragment.

Inhibitors of BACE1 can in addition be used to treat the following diseases: IBM (inclusion body myositis) (Vattemi G. et al., Lancet. 2001 Dec 8;358(9297):1962-4), Down's Syndrome (Barbiero L. et al, Exp Neurol. 2003 Aug;182(2):335-45), Wilson's Disease (Sugimoto I. et al., J Biol Chem. 2007 Nov 30;282(48):34896-903), Whipple's disease (Desnues B. et al., Clin Vaccine Immunol. 2006 Feb;13(2):170-8), SpinoCerebellar Ataxia 1 and SpinoCerebellar Ataxia 7 (Gatchel J.R. et al., Proc Natl Acad Sci U S A 2008 Jan 29;105(4):1291-6), Dermatomyositis (Greenberg S.A. et al., Ann Neurol. 2005 May;57(5):664-78 and Greenberg S.A. et al., Neurol 2005 May;57(5):664-78), Kaposi Sarcoma (Lagos D. et al, Blood, 2007 Feb 15; 109(4):1550-8), Glioblastoma multiforme (E-MEXP-2576, http://www.ebi.ac.uk/microarray-as/aer/result?queryFor=PhysicalArrayDesign&aAccession=A-MEXP-258), Rheumatoid arthritis (Ungethuem U. *et al*, GSE2053), Amyotrophic lateral sclerosis (Koistinen H. et al., Muscle Nerve. 2006 Oct;34(4):444-50 and Li Q.X. et al, Aging Cell. 2006 Apr;5(2):153-65), Huntington's Disease (Kim Y.J. et al., Neurobiol Dis. 2006 May;22(2):346-56. Epub 2006 Jan 19 and Hodges A. et al., Hum Mol Genet. 2006 Mar 15;15(6):965-77. Epub 2006 Feb 8), Multiple Mieloma (Kihara Y. et al, Proc Natl Acad Sci U S A. 2009 Dec 22;106(51):21807-12), Malignant melanoma (Talantov D. et al, Clin Cancer Res. 2005 Oct 15;11(20):7234-42), Sjogren syndrome (Basset C. et al., Scand J Immunol. 2000 Mar;51(3):307-11), Lupus erythematosus (Grewal P.K. et al, Mol Cell Biol. 2006, Jul;26(13):4970-81), Macrophagic myofasciitis, juvenile idiopathic arthritis, granulomatous arthritis, Breast cancer (Hedlund M. et al, Cancer Res. 2008 Jan 15;68(2):388-94 and Kondoh K. et al., Breast Cancer Res Treat. 2003 Mar;78(1):37-44), Gastrointestinal diseases (Hoffmeister A. et al, JOP. 2009 Sep 4;10(5):501-6), Autoimmune/inflammatory diseases (Woodard-Grice A.V. et al., J Biol Chem. 2008 Sep 26;283(39):26364-73. Epub 2008 Jul 23), Rheumatoid Arthritis (Toegel S. et al, Osteoarthritis Cartilage. 2010 Feb;18(2):240-8. Epub 2009 Sep 22), Inflammatory reactions (Lichtenthaler S.F. et al., J Biol Chem. 2003 Dec 5;278(49):48713-9. Epub 2003 Sep 24), Arterial Thrombosis (Merten M. et al., Z Kardiol. 2004 Nov;93(11):855-63), Cardiovascular diseases such as Myocardial infarction and stroke (Maugeri N. et al., Srp Arh Celok Lek. 2010 Jan;138 Suppl 1:50-2) and Graves disease (Kiljański J. et al, Thyroid. 2005 Jul;15(7):645-52).

WO 2014/166906, WO 2014/134341 and WO 2015/156421 describe oxazines and their use as BACE-1 inhibitors.

The present invention provides a novel compound of formula I, their manufacture, medicaments based on a compound in accordance with the invention and their production as well as the use of a compound of formula I in the control or prevention of illnesses such as Alzheimer's disease. Furthermore the use of a compound of formula I in the treatment of amyotrophic lateral sclerosis (ALS), arterial thrombosis, autoimmune/inflammatory diseases, cancer such as breast cancer, cardiovascular diseases such as myocardial infarction and stroke, dermatomyositis, Down's Syndrome, gastrointestinal diseases, Glioblastoma multiforme, Graves Disease, Huntington's Disease, inclusion body myositis (IBM), inflammatory reactions, Kaposi Sarcoma, Kostmann Disease, lupus erythematosus, macrophagic myofasciitis, juvenile idiopathic arthritis, granulomatous arthritis, malignant melanoma, multiple mieloma, rheumatoid arthritis, Sjogren syndrome, SpinoCerebellar Ataxia 1, SpinoCerebellar Ataxia 7, Whipple's Disease and Wilson's Disease. The novel compound of formula I has improved pharmacological properties.

### Field of the Invention

The present invention provides N-[6-[(4R,5R,6S)-2-amino-5-fluoro-4-methyl-6-(trifluoromethyl)-5,6-dihydro-1,3-oxazin-4-yl]-5-fluoro-2-pyridyl]-5-cyano-3-methyl-pyridine-2-carboxamide having BACE1 inhibitory properties, its manufacture, pharmaceutical compositions containing them and their use as therapeutically active substances.

### Summary of the Invention

The present invention provides a compound of formula I, 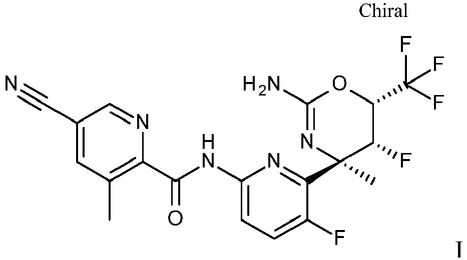 or a pharmaceutically acceptable salt thereof.

The present compound has Asp2 (β-secretase, BACE1 or Memapsin-2) inhibitory activity and may therefore be used in the therapeutic and/or prophylactic treatment of diseases and disorders characterized by elevated β-amyloid levels and/or β-amyloid oligomers and/or β-amyloid plaques, particularly Alzheimer's disease. Present compound and its pharmaceutically active salts has further several properties like e.g. a good bioavailability that makes it suitable for drug development.

### Detailed Description of the Invention

The present invention provides a compound of formula I and their pharmaceutically acceptable salts thereof, the preparation of the above mentioned compound, medicaments containing it and its manufacture as well as the use of the above mentioned compound in the therapeutic and/or prophylactic treatment of diseases and disorders which are associated with inhibition of BACE1, such as Alzheimer's disease. Furthermore, the formation, or formation and deposition, of β-amyloid plaques in, on or around neurological tissue (e.g., the brain) are inhibited by present compound by inhibiting the Aβ production from APP or an APP fragment.

The following definitions of the general terms used in the present description apply irrespectively of whether the terms in question appear alone or in combination with other groups.

Unless otherwise stated, the following terms used in this Application, including the specification and claims, have the definitions given below. It must be noted that, as used in the specification and the appended claims, the singular forms "a", "an," and "the" include plural referents unless the context clearly dictates otherwise.

The term "pharmaceutically acceptable salts" refers to salts that are suitable for use in contact with the tissues of humans and animals. Examples of suitable salts with inorganic and organic acids are, but are not limited to acetic acid, citric acid, formic acid, fumaric acid, hydrochloric acid, lactic acid, maleic acid, malic acid, methane-sulfonic acid, nitric acid, phosphoric acid, p-toluenesulphonic acid, succinic acid, sulfuric acid (sulphuric acid), tartaric acid, trifluoroacetic acid and the like. Particular acids are formic acid, trifluoroacetic acid and hydrochloric acid. Specific acids are hydrochloric acid, trifluoroacetic acid and fumaric acid.

The terms "pharmaceutically acceptable carrier" and "pharmaceutically acceptable auxiliary substance" refer to carriers and auxiliary substances such as diluents or excipients that are compatible with the other ingredients of the formulation.

The term "pharmaceutical composition" encompasses a product comprising specified ingredients in pre-determined amounts or proportions, as well as any product that results, directly or indirectly, from combining specified ingredients in specified amounts. Particularly it encompasses a product comprising one or more active ingredients, and an optional carrier comprising inert ingredients, as well as any product that results, directly or indirectly, from combination, complexation or aggregation of any two or more of the ingredients, or from dissociation of one or more of the ingredients, or from other types of reactions or interactions of one or more of the ingredients.

The term "inhibitor" denotes a compound which competes with, reduces or prevents the binding of a particular ligand to particular receptor or which reduces or prevents the inhibition of the function of a particular protein.

The term "half maximal inhibitory concentration" (IC₅₀) denotes the concentration of a particular compound required for obtaining 50% inhibition of a biological process in vitro. IC₅₀ values can be converted logarithmically to pIC₅₀ values (-log IC₅₀), in which higher values indicate exponentially greater potency. The IC₅₀ value is not an absolute value but depends on experimental conditions e.g. concentrations employed. The IC₅₀ value can be converted to an absolute inhibition constant (Ki) using the Cheng-Prusoff equation (Biochem. Pharmacol. (1973) 22:3099). The term "inhibition constant" (Ki) denotes the absolute binding affinity of a particular inhibitor to a receptor. It is measured using competition binding assays and is equal to the concentration where the particular inhibitor would occupy 50% of the receptors if no competing ligand (e.g. a radioligand) was present. Ki values can be converted logarithmically to pKi values (-log Ki), in which higher values indicate exponentially greater potency.

"Therapeutically effective amount" means an amount of a compound that, when administered to a subject for treating a disease state, is sufficient to effect such treatment for the disease state. The "therapeutically effective amount" will vary depending on the compound, disease state being treated, the severity or the disease treated, the age and relative health of the subject, the route and form of administration, the judgment of the attending medical or veterinary practitioner, and other factors.

The term "as defined herein" and "as described herein" when referring to a variable incorporates by reference the broad definition of the variable as well as particularly, more particularly and most particularly definitions, if any.

The terms "treating", "contacting" and "reacting" when referring to a chemical reaction means adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product.

The term "protecting group" denotes the group which selectively blocks a reactive site in a multifunctional compound such that a chemical reaction can be carried out selectively at another unprotected reactive site in the meaning conventionally associated with it in synthetic chemistry. Protecting groups can be removed at the appropriate point. Exemplary protecting groups are amino-protecting groups, carboxy-protecting groups or hydroxy-protecting groups. The term "amino protecting group" (here also X) denotes groups intended to protect an amino group and includes benzyl, benzyloxycarbonyl (carbobenzyloxy, CBZ), 9-Fluorenylmethyloxycarbonyl (FMOC), p-methoxybenzyloxycarbonyl, p-nitrobenzyloxycarbonyl, *tert*-butoxycarbonyl (BOC), and trifluoroacetyl. Further examples of these groups are found in T. W. Greene and P. G. M. Wuts, "Protective Groups in Organic Synthesis", 2nd ed., John Wiley & Sons, Inc., New York, NY, 1991, chapter 7; E. Haslam, "Protective Groups in Organic Chemistry", J. G. W. McOmie, Ed., Plenum Press, New York, NY, 1973, Chapter 5, and T.W. Greene, "Protective Groups in Organic Synthesis", John Wiley and Sons, New York, NY, 1981. The term "protected amino group" refers to an amino group substituted by an amino-protecting groups. Particular amino-protecting groups are *tert*-butoxycarbonyl group and dimethoxytrityl.

The term "leaving group" denotes the group with the meaning conventionally associated with it in synthetic organic chemistry, i.e., an atom or group displaceable under substitution reaction conditions. Examples of leaving groups include halogen, in particular bromo, alkane- or arylenesulfonyloxy, such as methanesulfonyloxy, ethanesulfonyloxy, thiomethyl, benzenesulfonyloxy, tosyloxy, dihalophosphinoyloxy, optionally substituted benzyloxy, isopropyloxy, and acyloxy.

The term "pharmaceutically acceptable excipient" denotes any ingredient having no therapeutic activity and being non-toxic such as disintegrators, binders, fillers, solvents, buffers, tonicity agents, stabilizers, antioxidants, surfactants or lubricants used in formulating pharmaceutical products.

Whenever a chiral carbon is present in a chemical structure, it is intended that all stereoisomers associated with that chiral carbon are encompassed by the structure as pure stereoisomers as well as mixtures thereof.

The invention also provides pharmaceutical compositions, methods of using, and methods of preparing the aforementioned compound.

All separate embodiments may be combined.

One embodiment of the invention provides a compound of formula I, 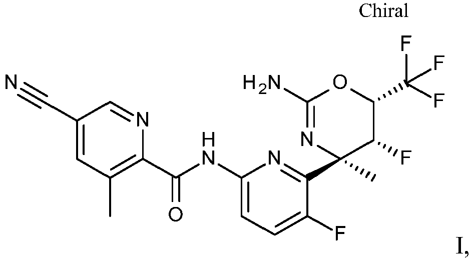 or pharmaceutically acceptable salts thereof.

A certain embodiment relates to N-[6-[(4R,5R,6S)-2-amino-5-fluoro-4-methyl-6-(trifluoromethyl)-5,6-dihydro-1,3-oxazin-4-yl]-5-fluoro-2-pyridyl]-5-cyano-3-methyl-pyridine-2-carboxamide.

Also disclosed is a metabolite M1 of a compound of formula I

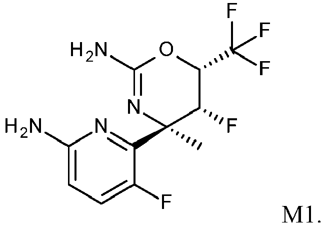

A certain embodiment relates to a compound of formula I as defined herein, which process comprises reacting a compound of formula XI' with a compound of formula XII' to a compound of formula I. 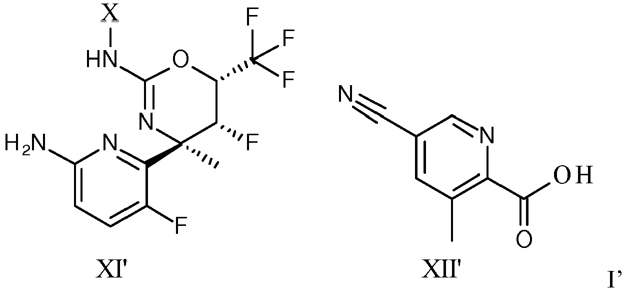 wherein X is an amino protecting group.

A certain embodiment of the invention provides a compound of formula I as described herein, whenever prepared by a process as defined above.

A certain embodiment of the invention provides a compound of formula I as described herein for use as therapeutically active substance.

A certain embodiment of the invention provides a compound of formula I as described herein for the use as inhibitor of BACE1 activity.

A certain embodiment of the invention provides a compound of formula I as described herein for the use as therapeutically active substance for the therapeutic and/or prophylactic treatment of diseases and disorders characterized by elevated β-amyloid levels and/or β-amyloid oligomers and/or β-amyloid plaques or Alzheimer's disease.

A certain embodiment of the invention provides a compound of formula I as described herein for the use as therapeutically active substance for the therapeutic and/or prophylactic treatment of Alzheimer's disease.

A certain embodiment of the invention provides a compound of formula I as described herein for the use as therapeutically active substance for the therapeutic and/or prophylactic treatment of amyotrophic lateral sclerosis (ALS), arterial thrombosis, autoimmune/inflammatory diseases, cancer such as breast cancer, cardiovascular diseases such as myocardial infarction and stroke, dermatomyositis, Down's Syndrome, gastrointestinal diseases, Glioblastoma multiforme, Graves Disease, Huntington's Disease, inclusion body myositis (IBM), inflammatory reactions, Kaposi Sarcoma, Kostmann Disease, lupus erythematosus, macrophagic myofasciitis, juvenile idiopathic arthritis, granulomatous arthritis, malignant melanoma, multiple mieloma, rheumatoid arthritis, Sjogren syndrome, SpinoCerebellar Ataxia 1, SpinoCerebellar Ataxia 7, Whipple's Disease or Wilson's Disease.

A certain embodiment of the invention provides a pharmaceutical composition comprising a compound of formula I as described herein and a pharmaceutically acceptable carrier and/or a pharmaceutically acceptable auxiliary substance.

A certain embodiment of the disclosure provides the use of a compound of formula I as described herein for the manufacture of a medicament for the use in inhibition of BACE1 activity.

A certain embodiment of the disclosure provides the use of a compound of formula I as described herein for the manufacture of a medicament for the therapeutic and/or prophylactic treatment of diseases and disorders characterized by elevated β-amyloid levels and/or β-amyloid oligomers and/or β-amyloid plaques or Alzheimer's disease.

A certain embodiment of the disclosure provides the use of a compound of formula I as described herein for the manufacture of a medicament for the therapeutic and/or prophylactic treatment of Alzheimer's disease.

A certain embodiment of the disclosure provides the use of a compound of formula I as described herein for the manufacture of a medicament for the therapeutic and/or prophylactic treatment of amyotrophic lateral sclerosis (ALS), arterial thrombosis, autoimmune/inflammatory diseases, cancer such as breast cancer, cardiovascular diseases such as myocardial infarction and stroke, dermatomyositis, Down's Syndrome, gastrointestinal diseases, Glioblastoma multiforme, Graves Disease, Huntington's Disease, inclusion body myositis (IBM), inflammatory reactions, Kaposi Sarcoma, Kostmann Disease, lupus erythematosus, macrophagic myofasciitis, juvenile idiopathic arthritis, granulomatous arthritis, malignant melanoma, multiple mieloma, rheumatoid arthritis, Sjogren syndrome, SpinoCerebellar Ataxia 1, SpinoCerebellar Ataxia 7, Whipple's Disease or Wilson's Disease.

A certain embodiment of the disclosure provides the use of a compound of formula I as described herein for the manufacture of a medicament for the therapeutic and/or prophylactic treatment of Alzheimer's disease.

A certain embodiment of the disclosure provides a compound of formula I as described herein for the use in inhibition of BACE1 activity.

A certain embodiment of the invention provides a compound of formula I as described herein for the use in the therapeutic and/or prophylactic treatment of diseases and disorders characterized by elevated β-amyloid levels and/or β-amyloid oligomers and/or β-amyloid plaques or Alzheimer's disease.

A certain embodiment of the invention provides a compound of formula I as described herein for the use in the therapeutic and/or prophylactic treatment of Alzheimer's disease.

A certain embodiment of the invention provides a compound of formula I as described herein for the use in the therapeutic and/or prophylactic treatment of amyotrophic lateral sclerosis (ALS), arterial thrombosis, autoimmune/inflammatory diseases, cancer such as breast cancer, cardiovascular diseases such as myocardial infarction and stroke, dermatomyositis, Down's Syndrome, gastrointestinal diseases, Glioblastoma multiforme, Graves Disease, Huntington's Disease, inclusion body myositis (IBM), inflammatory reactions, Kaposi Sarcoma, Kostmann Disease, lupus erythematosus, macrophagic myofasciitis, juvenile idiopathic arthritis, granulomatous arthritis, malignant melanoma, multiple mieloma, rheumatoid arthritis, Sjogren syndrome, SpinoCerebellar Ataxia 1, SpinoCerebellar Ataxia 7, Whipple's Disease or Wilson's Disease.

A certain embodiment of the disclosure provides a method for the use in inhibition of BACE1 activity, particularly for the therapeutic and/or prophylactic treatment of diseases and disorders characterized by elevated β-amyloid levels and/or β-amyloid oligomers and/or β-amyloid plaques or Alzheimer's disease, which method comprises administering compound of formula I as described herein to a human being or animal.

A certain embodiment of the disclosure provides a method for the use in the therapeutic and/or prophylactic treatment of Alzheimer's disease, which method comprises administering a compound of formula I as described herein to a human being or animal.

A certain embodiment of the disclosure provides a method for the use in the therapeutic and/or prophylactic treatment of amyotrophic lateral sclerosis (ALS), arterial thrombosis, autoimmune/inflammatory diseases, cancer such as breast cancer, cardiovascular diseases such as myocardial infarction and stroke, dermatomyositis, Down's Syndrome, gastrointestinal diseases, Glioblastoma multiforme, Graves Disease, Huntington's Disease, inclusion body myositis (IBM), inflammatory reactions, Kaposi Sarcoma, Kostmann Disease, lupus erythematosus, macrophagic myofasciitis, juvenile idiopathic arthritis, granulomatous arthritis, malignant melanoma, multiple mieloma, rheumatoid arthritis, Sjogren syndrome, SpinoCerebellar Ataxia 1, SpinoCerebellar Ataxia 7, Whipple's Disease or Wilson's Disease, which method comprises administering a compound of formula I as described herein to a human being or animal.

The skilled person in the art will recognize that the compound of formula I can exist in tautomeric form

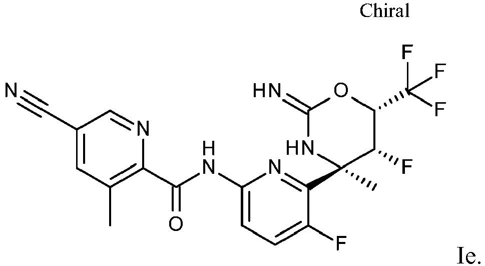

All tautomeric forms are encompassed in the present invention.

The compound of formula I may be prepared in accordance with the following schemes. The starting material is commercially available or may be prepared in accordance with known methods. Any previously defined residues and variables will continue to have the previously defined meaning unless otherwise indicated.

The optically pure enantiomer of Formula I means that the compound contains > 90 % of the desired isomer by weight, particularly > 95 % of the desired isomer by weight, or more particularly > 99 % of the desired isomer by weight, said weight percent based upon the total weight of the isomer(s) of the compound. Chirally pure or chirally enriched compound may be prepared by chirally selective synthesis or by separation of enantiomers. The separation of enantiomers may be carried out on the final product or alternatively on a suitable intermediate.

The compound of formula I may be prepared in accordance with the following scheme. The starting material may be prepared in accordance with known methods. Any previously defined residues and variables will continue to have the previously defined meaning unless otherwise indicated.

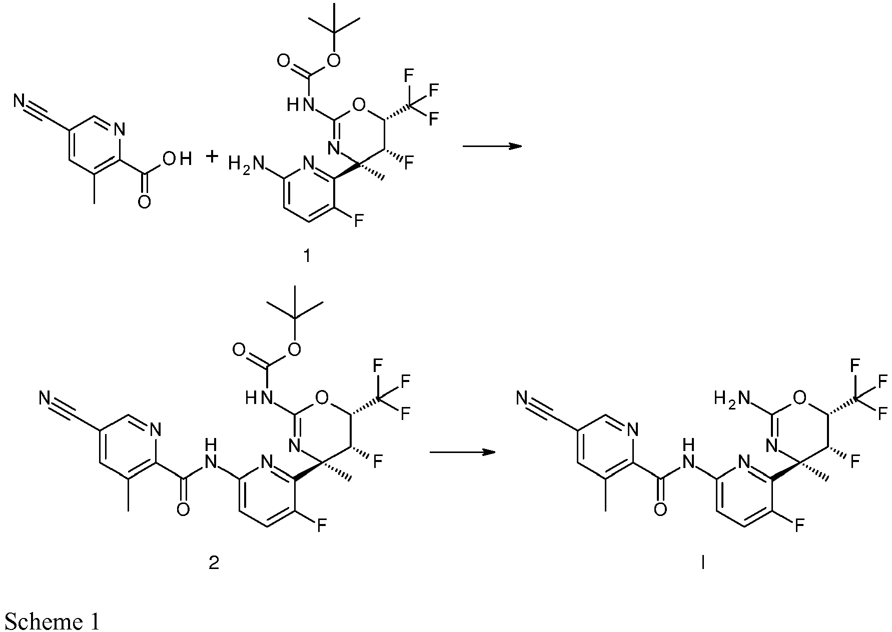

Compound of formula I can be prepared according to reaction scheme 1. Aromatic amine 1 (CAS 1630973-75-9; WO 2014166906) is reacted with 5-cyano-3-methyl-pyridine-2-carboxylic acid (CAS 1262860-49-0) in the presence of a suitable amide coupling reagent such as HATU, TBTU, BOP-Cl, oxalyl chloride, Ghosez's reagent, etc. in the presence of a suitable base such as DIEA, triethyl amine, etc. in an organic solvent such as acetonitrile, dichloro methane, THF, dioxane, DMF, DMA, NMP, etc. to form amide 2. The Boc-protecting group of compound 2 can be removed by using an appropriate acid such as TFA, formic acid, methylsulfonic acid etc. to form compound I.

The corresponding pharmaceutically acceptable salts with acids can be obtained by standard methods known to the person skilled in the art, e.g. by dissolving the compound of formula I in a suitable solvent such as e.g. dioxane or THF and adding an appropriate amount of the corresponding acid. The products can usually be isolated by filtration or by chromatography. The conversion of a compound of formula I into a pharmaceutically acceptable salt with a base can be carried out by treatment of such a compound with such a base. One possible method to form such a salt is e.g. by addition of 1/n equivalents of a basic salt such as e.g. M(OH)ₙ, wherein M = metal or ammonium cation and n = number of hydroxide anions, to a solution of the compound in a suitable solvent (e.g. ethanol, ethanol-water mixture, tetrahydrofuran-water mixture) and to remove the solvent by evaporation or lyophilization.

Insofar as their preparation is not described in the examples, the compound of formula I as well as all intermediate products can be prepared according to analogous methods or according to the methods set forth herein. Starting materials are commercially available, known in the art or can be prepared by methods known in the art or in analogy thereto.

It will be appreciated that the compound of general formula I in this invention may be derivatised at functional groups to provide derivatives which are capable of conversion back to the parent compound *in vivo.*

### Synthesis of Comparator Compounds 1, 2 and 3 (formula IIa/IIb)

In more detail, compounds of formula II according to the present invention can be prepared by the methods and procedures given below. Some typical procedures for the preparation of compounds of formula IIa and IIb are illustrated in scheme 1.

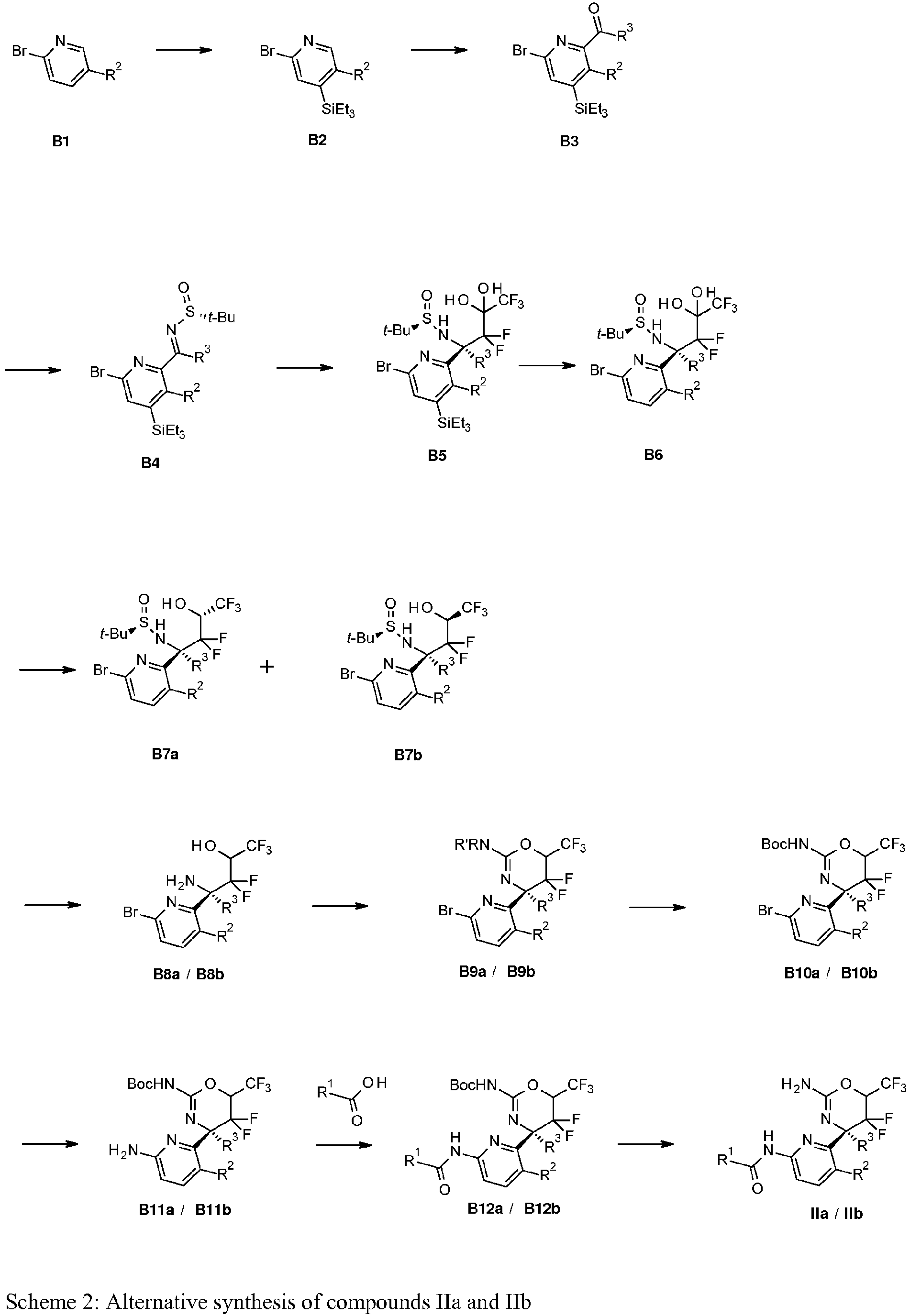

Non-commercial aryl ketones of general formula B3 can be synthesized from the silyl protected pyridine B2 prepared from pyridine B1 by reaction with a strong base, e.g. LDA and an alkylchlorosilane, preferably triethylchlorosilane in an inert aprotic solvents such as tetrahydrofuran or diethyl ether. The protected pyridine B2 can then be reacted again with a strong base, e.g. LDA and an amide, e.g. an acetamide for R³ = Me, preferably *N*,*N-*dimethylacetamide, in an inert aprotic solvents such as tetrahydrofuran or diethyl ether to give the desired aryl ketone B3.

Sulfinyl imines of formula B4 can be prepared in analogy to T.P. Tang & J.A. Ellman, J. Org. Chem. 1999, 64, 12, by condensation of an aryl ketone of formula B3 and a sulfinamide, e.g. an alkyl sulfinamide, most particularly (R)-*tert*-butylsulfinamide or (S)-*tert*-butylsulfinamide, in the presence of a Lewis acid such as e.g. a titanium(IV)alkoxide, more particularly titanium(IV)ethoxide, in a solvent such as an ether, e.g. diethyl ether or more particularly tetrahydrofuran.

The conversion of sulfinyl imines of formula B4 to pentafluoroketone hydrates of formula B5 proceeds stereoselectively by the chiral directing group as described by Tang & Ellman. The sulfinyl imines of formula B4 can be reacted with lithium pentafluoropropen-2-olate which can be generated by simple treatment of hexafluoroisopropanol with 2 eq. *n*-butyllithium as e.g. described in Org. Synth. 1999, 76, 151.

The conversion of the pentafluoroketone hydrates of formula B5 to the pentafluoroketone hydrates of formula B6 can be effected with tetrabutylammonium fluoride or preferably potassium fluoride in the presence of an acid e.g. acetic acid in an ether or an amide preferably in a mixture of THF and dimethylformamide at ambient to elevated temperature, particularly at 23 to 40 °C.

Pentafluoro alcohols of formula B7 can be prepared by the reduction of pentafluoroketone hydrates of formula B6 with an alkali hydride, e.g. lithium borohydride or sodium borohydride, preferably with sodium borohydride in a solvent mixture containing an ether, e.g. diethyl ether or more particularly tetrahydrofuran, and water, or an alcoholic solvent, such as methanol or ethanol, at temperatures between 0 °C and ambient temperature. The isomeric compounds of formula B7a and B7b can be separated by standard methods such as column chromatography on this stage.

Hydrolysis of the chiral directing group of formula B7a or B7b to give aminoalcohols of formula B8a or B8b can be accomplished with a mineral acid, e.g. sulfuric acid or particularly hydrochloric acid, in a solvent such as an ether, e.g. diethyl ether, tetrahydrofuran or more particularly 1,4-dioxane.

Aminooxazines of formula B9a or B9b (R, R'= H) can be prepared by reaction of aminoalcohols of formula B8a or B8b with cyanogen bromide in a solvent such as an alcohol, particularly ethanol.

The protection of the amino group of the 2-aminoxazine residue of formula B9a or B9b to produce compounds of general formula B10a or B10b, can be performed by reaction with di-tert-butyl dicarbonate under basic conditions, e.g. in the presence of an amine, such as triethylamine or diisopropylethylamine, in a solvent, such as tetrahydrofuran or dichloromethane, at temperatures between 0 to 40 °C, particular at ambient temperature.

Alternatively the compounds of general formula B10a or BlOb can be prepared by the following sequence: first, aminoalcohols of formula B8a or B8b are reacted with an isothiocyanate such as benzoylisothiocyanate (BzNCS) in solvents such as ethyl acetate, tetrahydrofuran or acetonitrile at temperatures between 0 °C and 80 °C, preferably 23 °C, affords the thiourea alcohols; second, the thiourea alcohols are cyclized to the *N*-benzoylated oxazines of formula B9a or B9b (R = H, R' = Bz) by dehydrosulfuration through reaction with a carbodiimide, like e.g. dicyclohexylcarbodiimide, diisopropylcarbodiimide or N-(3-dimethylaminopropyl)-*N'*-ethylcarbodiimide hydrochloride (EDC·HCl), preferably EDC·HCl, in solvents such as ethyl acetate, tetrahydrofuran or acetonitrile, preferably acetonitrile, at temperatures between 23 °C and 100 °C, preferably 80 °C; third, the switch of protecting groups from the *N*-benzoylated oxazines of formula B9a or B9b (R = H, R' = Bz) to the *N-tert-*butoxycarbonylated oxazines of formula B10a or BlOb can be achieved in a two-step procedure by first reaction with di-*tert*-butyldicarbonate (Boc₂O) in the presence of an amine base such as triethylamine or *N*-ethyl-*N,N*-diisopropylamine, in a solvent such as dichlormethane, tetrahydrofuran or acetonitrile, at temperatures between 0 °C and 40 °C, preferably 23 °C, to give the doubly acylated oxazine of formula B9a or B9b (R = Boc, R' = Bz), and second selective removal of the benzoyl group by reaction of the doubly acylated oxazine of formula B9a or B9b (R = Boc, R' = Bz) with an amine nucleophile, like e.g. diethylamine, dimethylamine or ammonia, preferably ammonia, in a solvent such as dichloromethane or tetrahydrofuran, preferably tetrahydrofuran, at temperatures between 0 °C and 40 °C, preferably 23 °C.

The conversion of the bromo group in formula B10a or BlOb to the amine group in formula B11a or B11b can be performed by reaction with an azide, in particular sodium azide and a cooper(I) halide in particular copper(I) iodide in the presence of L-ascorbate and an alkyl-1,2-diamine in particular trans-N,N'-dimethylcyclohexane-1,2-diamine in a protic solvent such as an alcohol in particular ethanol and water or a polar ether such as 1,4-dioxane and water at elevated temperature preferably approximately 70 °C.

The coupling of the aromatic amine B11a or B11B with carboxylic acids to give amides of formula B12a or B12b can be effected with T3P in an aprotic solvent such as EtOAc at ambient temperature; or alternatively the carboxylic acids can be activated by using reagents such as oxalyl chloride or 1-chloro-N,N,2-trimethyl-1-propenylamine (Ghosez's reagent, CAS-no. 26189-59-3) in a chlorinated solvent such as dichloromethane at 0 °C followed by reaction with the aromatic amine B11a or B11b in the presence of an amine base such as triethylamine or diisopropylethylamine at 0 °C to ambient temperature.

The cleavage of the protecting tert-butoxy carbonyl groups in compounds of formula B12a or B12b to produce compounds of general formula IIa or IIb can be effected by acid, such as trifluoroacetic acid, in inert solvents, such as dichloromethane, at temperatures between 0 °C and ambient temperature.

Synthesis of Comparators 4-5 are described in WO 2014/134341 and 6-7 in WO 2014/166906.

The corresponding pharmaceutically acceptable salts with acids can be obtained by standard methods known to the person skilled in the art, e.g. by dissolving the compound of formula I in a suitable solvent such as e.g. dioxane or tetrahydrofuran and adding an appropriate amount of the corresponding acid. The products can usually be isolated by filtration or by chromatography. The conversion of a compound of formula I into a pharmaceutically acceptable salt with a base can be carried out by treatment of such a compound with such a base. One possible method to form such a salt is e.g. by addition of 1/n equivalents of a basic salt such as e.g. M(OH)ₙ, wherein M = metal or ammonium cation and n = number of hydroxide anions, to a solution of the compound in a suitable solvent (e.g. ethanol, ethanol-water mixture, tetrahydrofuran-water mixture) and to remove the solvent by evaporation or lyophilisation. Particular salts are hydrochloride, formate and trifluoroacetate. A specific salt is trifluoroacetate.

### Pharmacological Tests

The compound of formula I and its pharmaceutically acceptable salts possess valuable pharmacological properties. It has been found that the compound of the present invention is associated with inhibition of BACE1 activity. The compound was investigated in accordance with the test given hereinafter.

### Cellular Aβ-lowering assay:

The Abeta 40 AlphaLISA Assay can be used. The HEK293 APP cells were seeded in 96 well Microtiter plates in cell culture medium (Iscove's, plus 10% (v/v) fetal bovine serum, penicillin/streptomycin) to about 80% confluency and the compounds were added at a 3x concentration in 1/3 volume of culture medium (final DMSO concentration was kept at 1 % v/v). After 18-20 hrs incubation at 37°C and 5% CO₂ in a humidified incubator, the culture supernatants were harvested for the determination of Aβ 40 concentrations using Perkin-Elmer Human Amyloid beta 1-40 (high specificity) Kit (Cat# AL275C).

In a Perkin-Elmer White Optiplate-384 (Cat# 6007290), 2ul culture supernatants were combined with 2µl of a 10X AlphaLISA Anti-hAβ Acceptor beads + Biotinylated Antibody Anti-Aβ 1-40 Mix (50 µg/mL / 5nM). After 1 hour room temperature incubation, 16µl of a 1.25 X preparation of Streptavidin (SA) Donor beads (25µg/mL) were added and incubated for 30 minutes in the Dark. Light Emission at 615 nm was then recorded using EnVision-Alpha Reader. Levels of Aβ 40 in the culture supernatants were calculated as percentage of maximum signal (cells treated with 1% DMSO without inhibitor). The IC₅₀ value were calculated using the Excel XLfit software.

### BACE1 cell-free assay (BACE1 Enzyme Assay)

The BACE1 cell-free IC50 assay was described in H. Hilpert et al., J. Med. Chem. 2013, 56, 3980-3995 (dx.doi.org/10.1021/jm400225m)

### Cellular Aβ-lowering assay (Cell-Based Assay)

The cellular Aβ-lowering IC50 assay was described in H. Hilpert et al., J. Med. Chem. 2013, 56, 3980-3995 (dx.doi.org/10.1021/jm400225m)

### BACE2 cell-free assay (BACE2 Enzyme Assay)

The BACE2 cell-free IC50 assay was described in H. Hilpert et al., J. Med. Chem. 2013, 56, 3980-3995 (dx.doi.org/10.1021/jm400225m)

### Mouse acute in vivo model (Inhibition of Aβ40 in Brain of Wild-Type Mice)

The acute in vivo model was described in H. Hilpert et al., J. Med. Chem. 2013, 56, 3980-3995 (dx.doi.org/10.1021/jm400225m)

### BACE1 K_{D} SPR assay

Surface Plasmon Resonance measurements were performed on a Biacore T200 instrument. Immobilization of BACE-1 was performed by standard amine coupling chemistry on a CM5 sensor to achieve protein surface density of ∼8000 pg/mm2 (RU). Immobilization was performed using the acetate buffer (25 mM Na acetate, 150 mM NaCl, 0.01% P20, 3 mM EDTA, pH 4.5) as the running buffer at 25°C. In the first step the carboxyl groups of CM5 sensor surface were transformed to the reactive succinimide esters by contacting the sensor surface 7 minutes with a solution of 0.2 M N-ethyl-N-dimethylaminopolycarboodiimide (EDC) and 0.05 M N-hydroxysuccinimide (NHS). After the activation, the sensor surface was contacted with (∼ 5 µgml-1 BACE1 protein solution in 10mM sodium acetate coupling buffer (pH 4.5) to reach desired protein surface density. Finally, the excess of activated carboxylic groups on the surface was quenched with ethanolamine (1 M, pH 8.5, 7 min).

Binding assays were performed at 18°C under the same buffer conditions as immobilization of the protein. Compounds were dissolved in DMSO as 10 mM stocks and diluted in running buffer to perform titration series (5 concentrations with dilution factor of 2) on a protein and reference (activated and deactivated CM5 sensor surface) surfaces in parallel. Active site small molecule ligand (CAS 883889-53-0) was used as a reference to monitor binding activity of BACE1 during the experiments.

The BACE1 target residence time was calculated using the equation published in (R. Copeland et al., Nature Reviews Drug Discovery, 2006 (5), 730-739.): t_{1/2} = 0.693/k_{off}

### hERG IC₂₀ assay

The methods to determine the hERG IC20 values was described in Industrializing electrophysiology: HT automated patch clamp on SyncroPatch® 96 using instant frozen cells. Polonchuk L., Methods Mol Biol. 2014;1183:81-92. (doi: 10.1007/978-1-4939-1096-0_5.) PMID:25023303

### hERG patch liner assay

The methods to determine the hERG IC20 patch liner values were described in Toward a New Gold Standard for Early Safety: Automated Temperature-Controlled hERG Test on the PatchLiner. Polonchuk L., Front Pharmacol. 2012 Jan 26;3:3. (doi: 10.3389/fphar.2012.00003. eCollection 2012.) PMID:22303293

### P-gp transport assay (human and mouse) (P-gp Assay)

The P-gp transport assays were described in H. Hilpert et al., J. Med. Chem. 2013, 56, 3980-3995 (dx.doi.org/10.1021/jm400225m)

### Rat single dose PK study (Inhibition of CSF Aβ40 in Wistar Rats)

The rat single dose PK study was described in H. Hilpert et al., J. Med. Chem. 2013, 56, 3980-3995 (dx.doi.org/10.1021/jm400225m) with slight modifications.

Aβ 40 concentration was determined without solid phase extraction following a modified protocol from Perkin-Elmer with a commercially available antibody against the N-terminal part of rat Aβ (252Q6, Invitrogen cat. No. AMB0062) and an in-house C-terminal specific anti-Aβ40 antibody (BAP-24, Brockhaus M. et al., J. Neuroreport, 1998, 9:1481-1486) bound to acceptor beads (PerkinElmer, Cat.6772002). 20 µl analyte per well (reconstituted extracts or standard) were loaded to a White Optiplate-96 (Perkin-Elmer, Cat# 6005290). Then, a mixture of biotinylated BAP24 antibody (1:2000 diluted at approximately 1nM final concentration) and antibody 252Q6 bound to AlphaLISA acceptor beads (Perkin-Elmer cat. No. 6772002 (1:100 diluted, 10 ug/mL final concentration) in 1x AlphaLISA buffer (Perkin-Elmer, AL000F) was added. After 1 hour room temperature incubation in the dark, 20 µL of a streptavidin coated donor beads dilution (40µg/mL) were added and incubated for 30 minutes in the dark. Light emission at 615 nm was recorded using an EnVision AlphaScreen Reader (Perkin-Elmer). Levels of Aβ 40 were calculated as percentage of the baseline samples from non-treated animals.

### Plasma preparation rats:

Blood is transferred into a labeled EDTA-K2 Eppendorf tube and the sample is mixed by inverting the tubes several times. Blood is stored at -20°C.

### Cynomolgus monkey (Macacaca fascicularis) single dose PK study and Plasma Aβ40 determination

### Animal handling:

Blood and plasma collection will always be performed in the treatment cage. The animal care keeper grabs either one arm or one leg through the bars. The hairs are shaved for better visibility of the vein. The blood vessel is squeezed, the vein is punctured by using a butterfly and blood is taken. Once enough blood has been withdrawn a cotton swab is placed over the injection site and the needle is gently removed. Bleeding is stopped by pressing a cotton swab on the wound. Plasma samples were taken between 5 min and 48h.

### Plasma preparation cynomolgus

Blood is transferred into a labeled EDTA-K2 Eppendorf tube and the sample is mixed by inverting the tubes several times. Blood is stored at -20°C.
Aβ determination in cynomolgous plasma samples was done after solid phase extraction (Lanz, T. et al., J. of Neuroscience Methods, 2006, 157:71-81). Frozen plasma samples were thawed in a 25°C waterbath. 100uL plasma was added to 900ul 0.2% DEA aqueous solution (Sigma D8885) with 50mM NaCl containing complete protease inhibitor (Roche 05056489001) followed by incubation for 30 minutes at room temperature. 60 mg Oasis HLB LP 96-well plates (Waters, Milford Massachusetts, USA; Cat.186000679) were placed into extraction plate manifolds (Waters, Milford Massachusetts, USA) and connected to vacuum. Column plates were activated with 1 mL methanol (MeOH), followed by 1 mL distilled H2O. DEA-extracted samples were loaded and washed sequentially with 1 mL volume of 5 % and 30% MeOH and eluted with 0.8ml 2% NH4OH in 90% MeOH. Eluted samples were collected in Clavepak 96 racked 1.1 mL tubes (Denville Scientific), and dried over a N2 flow in a Caliper Turbovap96. Once samples were dried, they were stored at -80°C or measured immediately. Dried samples were reconstituted in 30ul AlphaLISA buffer.

Aβ concentration was determined following a modified protocol from Perkin-Elmer with a commercially available antibody against the N-terminal part of cynomolgus Aβ (6E10, Signet IG-39340) and an in-house C-terminal specific anti-Aβ40 antibody (BAP-24, Brockhaus M. et al., J. Neuroreport, 1998, 9:1481-1486). 20 µl analyte per well (reconstituted extracts or standard) were loaded to a White Optiplate-96 (Perkin-Elmer, Cat# 6005290). Then, a mixture of biotinylated 6E10 antibody (1:2000 diluted at approximately 1nM final concentration) and BAP24 bound to AlphaLISA acceptor beads (Perkin-Elmer cat. No. 6772002 (1:100 diluted, 10 ug/mL final concentration) in 1x AlphaLISA buffer (Perkin-Elmer, AL000F) was added. After 1 hour room temperature incubation in the dark, 20 µL of a streptavidin coated donor beads dilution (40µg/mL) were added and incubated for 30 minutes in the dark. Light emission at 615 nm was recorded using an EnVision AlphaScreen Reader (Perkin-Elmer). Levels of Aβ 40 were calculated as percentage of the baseline samples from non-treated animals.

**Table 1: IC₅₀ value**

| **Cmpd** | **Structure** | **BACE1** cell act. Aβ40 IC₅₀ [nM] | **BACE1** cell free IC₅₀ [nM] | **BACE2/BACE1** IC₅₀ ratio |
|---|---|---|---|---|
| Example 1 | 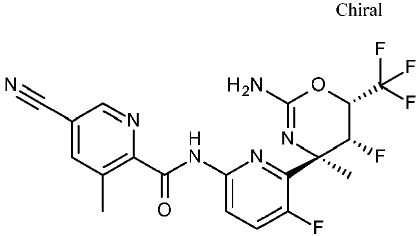 | 15 | 36 | 2.0 |
| Comparator 1 | 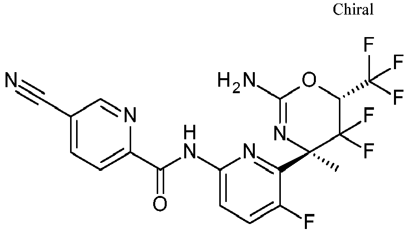 | 324 | 29 | 3.0 |
| Comparator 2 | 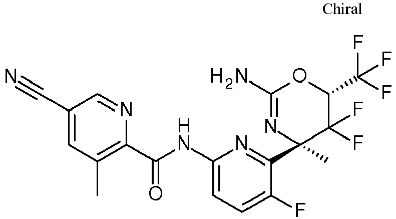 | 203 | 19 | 3.0 |
| Comparator 3 | 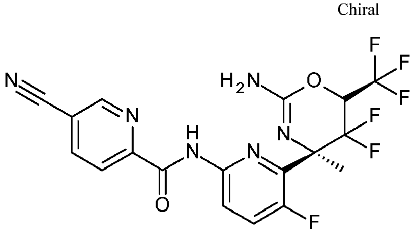 | 37400 | n.d. | n.d. |
| Comparator 4 Ex.70 of WO 2014/134341 | 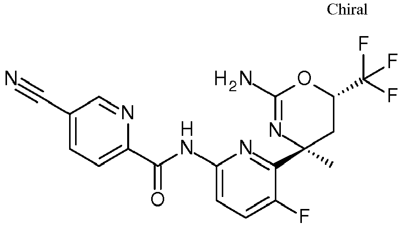 | 4 | 33 | 0.7 |
| Comparator 5 Ex.84 of WO 2014/134341 | 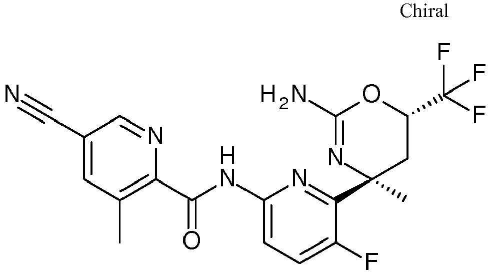 | 4 | 39 | 0.6 |
| Comparator 6 Ex.12 of WO 2014/166906 | 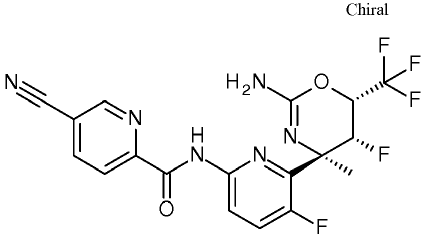 | 10 | 34 | 2.1 |
| Comparator 7 Ex.12 of WO 2014/166906 | 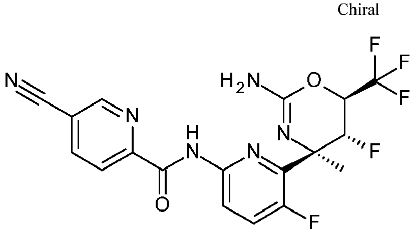 | 96 | 67 | 1.9 |

The determination of BACE1 activity can be either measured by using an enzymatic cell-free fluorescence assay (BACE1 cell free, table 2) or by a whole cell assay using HEK293 APP cells (Cellular Aβ-lowering, table 2). Both assays are suitable to determine BACE1 inhibition. As shown in table 1, example 1 shows in both assays potent inhibition with IC₅₀ values of 36 nM and 15 nM, respectively. Surprisingly, compounds containing one additional fluorine atom at the oxazine moiety such as comparators 1,2 and 3 display a significant lower efficacy in the cellular Aβ-lowering assay, being 13-2500-fold less active than example 1.

These compounds demonstrate an efficacy in the cellular Aβ-lowering assay well above an IC₅₀ of 100 nM, which is considered by a person skilled in the art as a threshold for viable drug candidates. Thus, beside a trifluoromethyl group attached to the oxazine ring, only one additional fluorine atom is tolerated for a drug candidate to be viable in that field.

Furthermore, comparator 3 and 7 demonstrate that the stereochemistry of the trifluoromethyl group plays a surprisingly crucial role. In example 1, the 6S-configured trifluoromethyl group gives rise to high potency, whereas the 6R-configured comparators 3 and 7 lead to significant lower efficacies in the cellular Aβ-lowering assay, being 6.4 - 2500-fold less active, with comparator 7 being close to the threshold of 100 nM activity for a compound regarded suitable for further development as pharmaceutical drug.

When comparing the close structural analogs comparator 6 and comparator 7, which differ in their 6S- (comparator 6) or 6R-configuration (comparator 7) of the trifluoromethyl group, this effect is even more pronounced reaching nearly one order of magnitude.

Selectivity versus related targets is important in terms of safety of a drug candidate. BACE2 is a close homolog of BACE1. BACE2 was recently reported to play a crucial role in pigmentation of the skin by processing pigment cell-specific melanocyte protein (PMEL). (Proc Natl Acad Sci U S A. 2013 Jun 25;110(26):10658-63. doi: 10.1073/pnas.1220748110. Epub 2013 Jun 10). It is believed that PMEL contributes in melanogenesis. Therefore, selectivity versus BACE2 is advantageous when it comes to pigmentation of the skin. The selectivity ratio of BACE2/BACE1 IC₅₀ was calculated by dividing the BACE2 IC₅₀ value by the BACE1 IC₅₀ value determined each in the cell free assay. As shown in table 1, example 1 has a 2-fold selectivity versus BACE2. Surprisingly, it was found that comparators 4 as well as 5 are more potent for BACE2 inhibition than for BACE1 inhibition with BACE2/BACE1 IC₅₀ ratios of 0.7 and 0.6, respectively. This implies a 3-fold improvement of example 1 compared to comparators 4 and 5.

### Single dose efficacy in mice

The mouse single dose efficacy was measured in brain homogenate from C57B1/6J wildtype mice after DEA extraction as described in H. Hilpert et al., J. Med. Chem. 2013, 56, 3980-3995 (dx.doi.org/10.1021/jm400225m).

Aβ 40 concentration was determined after solid phase extraction as described above and following a modified protocol from Perkin-Elmer with a commercially available antibody against the N-terminal part of rat Aβ (252Q6, Invitrogen cat. No. AMB0062) and an in-house C-terminal specific anti-Aβ40 antibody (BAP-24, Brockhaus M. et al., J. Neuroreport, 1998, 9:1481-1486) bound to acceptor beads (PerkinElmer, Cat.6772002). 20 µl analyte per well (reconstituted extracts or standard) were loaded to a White Optiplate-96 (Perkin-Elmer, Cat# 6005290). Then, a mixture of biotinylated BAP24 antibody (1:2000 diluted at approximately 1nM final concentration) and antibody 252Q6 bound to AlphaLISA acceptor beads (Perkin-Elmer cat. No. 6772002 (1:100 diluted, 10 ug/mL final concentration) in 1x AlphaLISA buffer (Perkin-Elmer, AL000F) was added. After 1 hour room temperature incubation in the dark, 20 uL of a streptavidin coated donor beads dilution (40µg/mL) were added and incubated for 30 minutes in the dark. Light emission at 615 nm was recorded using an EnVision AlphaScreen Reader (Perkin-Elmer). Levels of Aβ40 were calculated as percentage of the baseline samples from non-treated animals.

**Table 2: Acute in vivo efficacy after single p.o. dose in mice at 4 h post dose.**

| **Compound** | **Brain Aβ-lowering 30 mg/kg** | **Brain Aβ-lowering 10 mg/kg** | **Brain Aβ-lowering 3 mg/kg** |
|---|---|---|---|
| Example 1 | n.d. | 92% | 58% |
| Comparator 1 | 66% | 4% | 2% |
| Comparator 2 | n.d. | 42% | 10% |
| Comparator 3 | n.d. | n.d. | n.d. |
| Comparator 4 | n.d. | 75% | 33% |
| Comparator 5 | n.d. | 70% | 7% |
| Comparator 6 | n.d. | 94% | 86% |
| Comparator 7 | 70% | n.d. | n.d. |

Aβ40 lowering was determined after oral administration in wild-type mice (C57Bl/6J mice, n = 3-4) 4 h post dosing. Wild-type mice were used as they have a normal physiological level of APP expression and are thus expected to provide a better physiological model for Aβ-peptide production than APP-transgenic organisms. In vivo efficacy is a key factor for appropriate drug candidates, being more relevant than in vitro activities.

Example 1 shows a surprisingly high inhibition of Aβ40 production in the brain of mice in a dose-dependent manner. At a dose of 10 mg/kg per oz a 92% reduction of Aβ40 was observed. At 3 mg/kg 58% reduction of Aβ40 was observed. In stark contrast comparators 1 and 2 did only show a very weak reduction of Aβ40 of 2-10% at a dose of 3 mg/kg. Comparator 3 was not tested in vivo due to its very poor cellular potency of >35µM making it highly unlikely to be active in vivo. Surprisingly, comparator 4, and particularly the close analog to example 1, comparator 5 are significantly less active with 33% and 7% respectively, reduction of Aβ40 at 3 mg/kg than example 1. Based on the in vitro potency listed in table 2 for comparators 4 and 5, this result is surprising. Comparator 7 is less active at a dose of 30 mg/kg with 70% reduction of Aβ40 than example 1 at 10 mg/kg, confirming the surprising importance of the approriate stereochemistry of the trifluoromethyl group of the oxazine ring.

**Table 3: Dissociation constant K_{D} BACE1 and BACE1 target residence time values determined by Surface Plasmon Resonance**

| **Compound** | **K_{D} BACE1 [nM]** | **BACE1 residence time t_{1/2} [min]** |
|---|---|---|
| Example 1 | 2.0 | 16 |
| Comparator 1 | n.d. | n.d. |
| Comparator 2 | n.d. | n.d. |
| Comparator 3 | n.d. | n.d. |
| Comparator 4 | n.d. | n.d. |
| Comparator 5 | n.d. | n.d. |
| Comparator 6 | 6.3 | 4.4 |
| Comparator 7 | n.d. | n.d. |

Dissociation constants and target residence time are important aspects for pharmacological effects and target selectivity (R. Copeland et al., Nature Reviews Drug Discovery, 2006 (5), 730-739.). A low dissociation constant K_{D} and particularly a longer target residence time is expected to lead to a sustained pharmacological effect in vivo. A longer target residence time is thus preferred in drug development. Surprisingly, example 1 binds more tightly to BACE1 than comparator 6 with a K_{D} value of 2.0 nM versus 6.3 nM, respectively. The tight binding of example 1 is also reflected in the longer residence time on BACE1 with 16 minutes. In contrast, comparator 6 has a ∼4-fold shorter target residence time on BACE1.

**Table 4: hERG values and human/mouse P-gp efflux ratios ER**

| **Compound** | **hERG IC₂₀ [nM]** | **Human P-gp ER** | **Mouse P-gp ER** |
|---|---|---|---|
| Example 1 | >1000 | 1.7 | 3.5 |
| Comparator 1 | n.d. | 1.4 | 1.3 |
| Comparator 2 | n.d. | n.d. | 1.4 |
| Comparator 3 | n.d. | n.d. | n.d. |
| Comparator 4 | 40 | 1.9 | 3.9 |
| Comparator 5 | 440 | 3.3 | 8.1 |
| Comparator 6 | 400 | 1.1 | 2.4 |
| Comparator 7 | n.d. | n.d. | n.d. |

QT prolongation is a cardiovascular risk caused by drug candidates which inhibit the hERG channel (human ether a go-go). A sufficient safety margin between the hERG IC₂₀ value and the free plasma concentration necessary to achieve the envisaged thereapeutic effects is desired (ICH. The nonclinical evaluation of the potential for delayed ventricular repolarization (QT interval prolongation) by human pharmaceuticals (S7B), issued as CPMP/ICH/423/02, adopted by CHMP in May 2005 (http://www.ich.org/cache/compo/276-254-1.html; Redfern WS, Carlsson L, Davis AS, Lynch WG, MacKenzie I, Palethorpe S, Siegl PK, Strang I, Sullivan AT, Wallis R, Camm AJ, Hammond TG. Relationships between preclinical cardiac electrophysiology, clinical QT interval prolongation and torsade de pointes for a broad range of drugs: evidence for a provisional safety margin in drug development. Cardiovasc Res. 2003 Apr 1;58(1):32-45.)). Reducing the activity against efflux transporters expressed at the blood-brain-barrier such as P-glycoprotein P-gp, might result in reduction of the free plasma concentration by optimizing brain penetration.

In the assay of the P-gp efflux ratio ER as described hereinabove, a compound with ER < 2.5 is regarded most suitable. In addition, compounds with low hERG channel binding are suitable as well.

Example 1 in table 5 shows surprisingly favorable values for hERG IC₂₀ assay with >1000 nM. Further assessment in the hERG patchliner assay revealed an IC₂₀ value of 2340 nM for example 1. Example 1 also showed a favorable value for the human P-gp ER of 1.7.

Comaprator 4 in contrast binds very strongly to hERG with an IC₂₀ value of 40 nM. This might indicate a very high risk of strong QT prolongation at therapeutic concentrations. P-gp of comparator 4 is in a favorable range with ER 1.9. Comparator 5 shows a 5.3-fold higher affinity to hERG than example 1 and has in addition a 2-fold higher P-gp efflux ratio. The combination of both assays further stresses example 1 as suitable for development compared to comparator 5. Comparator 6 has as well a 6-fold higher affinity to hERG as compared to example 1, whereas P-gp ER is in the favorable range with 1.1.

**Table 5: Rat single dose PK study**

| **Compound** | **Oral Bioavailability F** | **Plasma concentration at 4 h [ng/mL]** | **CSF Aβ-lowering 1 mg/kg at 4 h** | **hERG safety margin** |
|---|---|---|---|---|
| Example 1 | 62% | 41 (5.7 free) (90.2 nM; 12.5 nM free) | 80% | 187-fold (hERG IC20/free plasma conc.) |
| Comparator 1 | n.d. | n.d. | n.d. | |
| Comparator 2 | n.d. | n.d. | n.d. | |
| Comparator 3 | n.d. | n.d. | n.d. | |
| Comparator 4 | n.d. | n.d. | n.d. | |
| Comparator 5 | n.d. | n.d. | n.d. | |
| Comparator 6 | 19% | 85 (16 free) (193 nM; 36.3 nM free) | 77% | 11-fold (hERG IC20/free plasma conc.) |
| Comparator 7 | n.d. | n.d. | n.d. | |

Table 5 shows the rat PK data and Aβ40-lowering in rat CSF at 4 h after administering the compound at 1 mg/kg. Surprisingly, example 1 showed a good oral bioavailability of 62% and a strong in vivo efficacy of 80% reduction of Aβ40 in rat CSF after 4 h post dose. The plasma concentration at 4 h was measured to be 90 nM, the free, unbound concentration was 12.5 nM. The hERG safety margin can be calculated using the hERG IC₂₀ value divided by the free plasma concentration. Example 1 has a safety margin of 187-fold, calculated with the IC₂₀ value of the hERG patchliner assay. Comparator 6 only demonstrated a low oral bioavailability of 19 % when dosed at 1 mg/kg. The free plasma concentration was measured to be 36.3 nM at 4 h post dose leading to a 77% reduction of Aβ40 in rat CSF. This value is in a similar range than for example 1, therefore calculation of the hERG safety margin can be performed equally. Comparator 6 only showed a 11-fold hERG safety margin. Example 1 has thus a 17-fold improvement of the safety margin at comparable efficacy levels of approximately 80% reduction of Aβ40 in rat CSF.

**Table 6: Cynomolgus monkey (Macacaca fascicularis) single dose PK study and Plasma Aβ40 determination**

| **Compound** | **Oral Bio-availability F** | **CL [ml/min/kg]** | **t_{1/2} p.o. [h]** | **AUC(0-24)/D p.o. [h*kg*ng/ml/mg]** | **Amide Hydro-lysis, M1 formation** | **Plasma Aβ-lowering 0.2 mg/kg at 24 h / 48 h** |
|---|---|---|---|---|---|---|
| Example 1 | 52% | 2.1 | 19 | 4160 | 1-2% | 57% / 50% |
| Comparator 1 | n.d. | n.d. | n.d. | n.d. | n.d. | n.d. |
| Comparator 2 | n.d. | n.d. | n.d. | n.d. | n.d. | n.d. |
| Comparator 3 | n.d. | n.d. | n.d. | n.d. | n.d. | n.d. |
| Comparator 4 | n.d. | n.d. | n.d. | n.d. | n.d. | n.d. |
| Comparator 5 | n.d. | n.d. | n.d. | n.d. | n.d. | n.d. |
| Comparator 6 | 63% | 9.7 | 11 | 840 | 20% | n.d. |
| Comparator 7 | n.d. | n.d. | n.d. | n.d. | n.d. | n.d. |

Higher species PK studies such as PK studies in cynomolgus monkey are a tool in predicting PK properties of a drug candidate in humans (Ward, K. W. and B. R. Smith (2004). "A Comprehensive Quantitative And Qualitative Evaluation Of Extrapolation Of Intravenous Pharmacokinetic Parameters From Rat, Dog, And Monkey To Humans. I. Clearance." DMD 32: 603-611). Example 1 and comparator 6 show both medium oral bioavailability of 52% and 63%, respectively. Surprisingly, example 1 demonstrated a low clearance in monkeys of 2.1 ml/min/kg associated with a long half-life of 19 h and a high plasma exposure AUC (0-24) dose normalized of 4160 h^{∗}kg^{∗}ng/ml/mg. Furthermore, a sustained plasma Aβ40-lowering of 57% (24 h post dose) and 50% (48 h post dose) was observed. In contrast, comparator 6 showed a significantly higher clearance of 9.7 ml/min/kg which translates into a much shorter half-life of 11 h and a 5-fold lower exposure when compared to example 1.

When analyzing metabolites of example 1 and comparator 6 it could be shown M1 (scheme 3) is formed by hydrolysis of the amide bond of both compounds. Surprisingly, formation of M1 in cynomolgus monkey PK studies was observed only to 1-2% of the total amount of example 1. In contrast, formation of M1 is occuring to 20% of the total amount of comparator 6. Blocking amide hydrolysis will lead to a lower clearance, longer half-life and a sustained pharmacological effect which is actually the case for example 1.

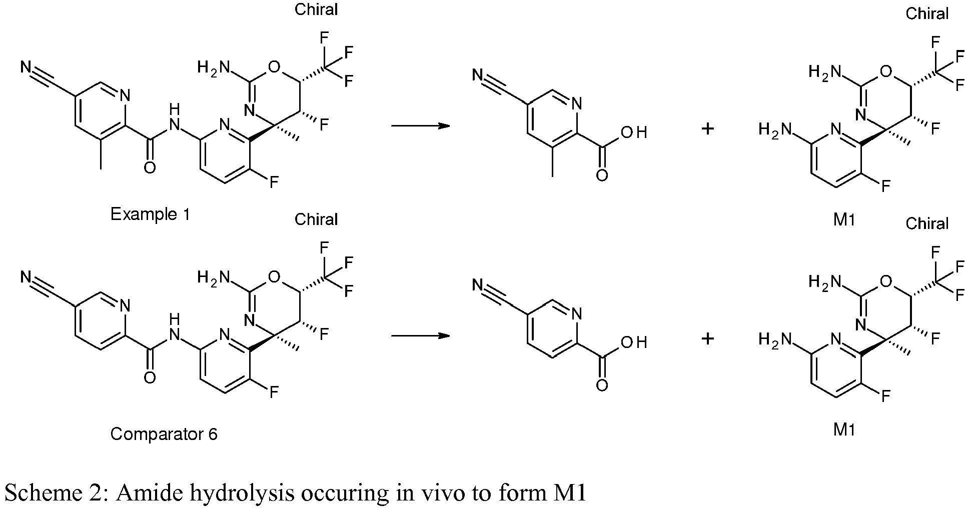

### Pharmaceutical Compositions

The compound of formula I and the pharmaceutically acceptable salts can be used as therapeutically active substances, e.g. in the form of pharmaceutical preparations. The pharmaceutical preparations can be administered orally, e.g. in the form of tablets, coated tablets, dragées, hard and soft gelatin capsules, solutions, emulsions or suspensions. The administration can, however, also be effected rectally, e.g. in the form of suppositories, or parenterally, e.g. in the form of injection solutions.

The compound of formula I and the pharmaceutically acceptable salts thereof can be processed with pharmaceutically inert, inorganic or organic carriers for the production of pharmaceutical preparations. Lactose, corn starch or derivatives thereof, talc, stearic acids or its salts and the like can be used, for example, as such carriers for tablets, coated tablets, dragées and hard gelatin capsules. Suitable carriers for soft gelatin capsules are, for example, vegetable oils, waxes, fats, semi-solid and liquid polyols and the like. Depending on the nature of the active substance no carriers are however usually required in the case of soft gelatin capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparations can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula I or a pharmaceutically acceptable salt thereof and a therapeutically inert carrier are also provided by the present invention, as is a process for their production, which comprises bringing one or more compounds of formula I and/or pharmaceutically acceptable salts thereof and, if desired, one or more other therapeutically valuable substances into a galenical administration form together with one or more therapeutically inert carriers.

The dosage can vary within wide limits and will, of course, have to be adjusted to the individual requirements in each particular case. In the case of oral administration the dosage for adults can vary from about 0.01 mg to about 1000 mg per day of a compound of general formula I or of the corresponding amount of a pharmaceutically acceptable salt thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical preparations conveniently contain about 1-500 mg, particularly 1-100 mg, of a compound of formula I. Examples of compositions according to the invention are:

### Example A

Tablets of the following composition are manufactured in the usual manner:

**Table 7: possible tablet composition**

| ingredient | mg/tablet | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

### Example B-1

Capsules of the following composition are manufactured:

**Table 8: possible capsule ingredient composition**

| ingredient | mg/capsule | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula I, lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 9: possible soft gelatin capsule ingredient composition**

| ingredient | mg/ capsule |
|---|---|
| Compound of formula I | 5 |
| Yellow wax | 8 |
| Hydrogenated Soya bean oil | 8 |
| Partially hydrogenated plant oils | 34 |
| Soya bean oil | 110 |
| Total | 165 |

**Table 10: possible soft gelatin capsule composition**

| ingredient | mg/ capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula I is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 11: possible suppository composition**

| ingredient | mg/supp. |
|---|---|
| Compound of formula I | 15 |
| Suppository mass | 1285 |
| Total | 1300 |

### Manufacturing Procedure

The suppository mass is melted in a glass or steel vessel, mixed thoroughly and cooled to 45°C. Thereupon, the finely powdered compound of formula I is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 12: possible injection solution composition**

| ingredient | mg/injection solution. |
|---|---|
| Compound of formula I | 3 |
| Polyethylene Glycol 400 | 150 |
| acetic acid | q.s. ad pH 5.0 |
| water for injection solutions | ad 1.0 ml |

### Manufacturing Procedure

The compound of formula I is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 13: possible sachet composition**

| ingredient | mg/sachet |
|---|---|
| Compound of formula I | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula I is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Experimental Part

The following examples are provided for illustration of the invention. They should not be considered as limiting the scope of the invention, but merely as being representative thereof.

**NMR:** ¹H NMR spectra were recorded on a Bruker AC-300 spectrometer at 25 °C with TMS (tetramethylsilane) or residual ¹H of the given deuterated solvents as internal standards.

MS: Mass spectra (MS) were measured either with ion spray positive or negative (ISP or ISN) method on a Perkin-Elmer SCIEX API 300 or with electron impact method (EI, 70 eV) on a Finnigan MAT SSQ 7000 spectrometer.

LC-MS (ESI, positive or negative ion) data were recorded on Waters UPLC-MS Systems equipped with Waters Acquity, a CTC PAL auto sampler and a Waters SQD single quadrupole mass spectrometer using ES ionization modes (positive and/or negative). The separation was achieved on a Zorbax Eclipse Plus C18 1,7 µm 2.1×30 mm column at 50 °C; A = 0.01% formic acid in water, B = acetonitrile at flow 1; gradient: 0 min 3% B, 0.2 min 3% B, 2 min 97% B, 1.7 min 97% B, 2.0 min 97% B. The injection volume was 2 µL. MS (ESI, positive or negative ion): FIA (flow injection analysis)-MS were recorded on an AppliedBiosystem API150 mass spectrometer. Sample introduction was made with a CTC PAL auto sampler and a Shimadzu LC-10ADVP Pump. The samples were directly flushed to the ESI source of the mass spectrometer with a flow 50µL/min of a mixture of acetonitrile and 10 mM ammonium acetate (1:1) without a column. The injection volume was 2 µL.

### Example 1

### N-(6-((4R,5R,6S)-2-amino-5-fluoro-4-methyl-6-(trifluoromethyl)-5,6-dihydro-4H-1,3-oxazin-4-yl)-5-fluoropyridin-2-yl)-5-cyano-3-methylpicolinamide

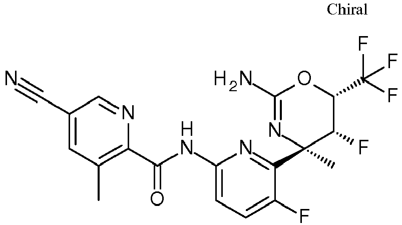

### Example 1a: tert-butyl N-[(4R,5R,6S)-4-[6-[(5-cyano-3-methyl-pyridine-2-carbonyl)amino]-3-fluoro-2-pyridyl]-5-fluoro-4-methyl-6-(trifluoromethyl)-5,6-dihydro-1,3-oxazin-2-yl] carbamate

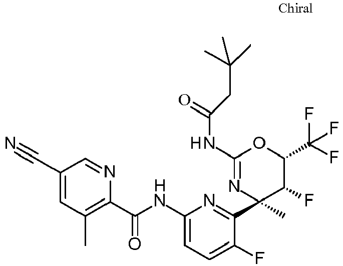

To a suspension of 5-cyano-3-methyl-pyridine-2-carboxylic acid (CAS 1262860-49-0) (38.7 mg, 239 µmol) in CH₂Cl₂ (560 µl) was added at 0 °C 1-chloro-N,N-2-trimethylprop-1-en-1-amine (Ghosez's reagent) (34.2 mg, 33.9 µl, 256 µmοl). The formed colorless solution was stirred at 0 °C for 15 min. A solution of tert-butyl ((4R,5R,6S)-4-(6-amino-3-fluoropyridin-2-yl)-5-fluoro-4-methyl-6-(trifluoromethyl)-5,6-dihydro-4H-1,3-oxazin-2-yl)carbamate (CAS 1630973-75-9; WO 2014166906) (0.07 g, 171 µmol) in CH₂Cl₂ (1.1 mL) and triethylamine (51.8 mg, 71.3 µL, 512 µmol) was added to the reaction mixture at 0 °C. The resulting light yellow solution was allowed to warm to 25 °C and stirred for additional 60 min. The reaction mixture was diluted with CH₂Cl₂ (3 mL) and extracted with saturated aqueous NaHCO₃ solution (2 x 1.5 mL). The aqueous layer was back-extracted with EtOAc (3 x 2 mL). The organic layers were combined, washed with saturated aqueous NaCl solution (1 x 2 mL), dried over Na₂SO₄ and concentrated in vacuo. The residue was purified two times by flash chromatography (silica gel, 4 g, 10% to 50% EtOAc in n-heptane) to yiled example 1a as a white solid (44 mg; 47 %). m/z = 555.3 [M+H]⁺, 1H NMR in CDCl3 is consistent with desired product.

### Example 1: N-(6-((4R,5R,6S)-2-amino-5-fluoro-4-methyl-6-(trifluoromethyl)-5,6-dihydro-4H-1,3-oxazin-4-yl)-5-fluoropyridin-2-yl)-5-cyano-3-methylpicolinamide

To a stirring solution of tert-butyl ((4R,5R,6S)-4-(6-(5-cyano-3-methylpicolinamido)-3-fluoropyridin-2-yl)-5-fluoro-4-methyl-6-(trifluoromethyl)-5,6-dihydro-4H-1,3-oxazin-2-yl)carbamate (0.044 g, 79.4 µmol) (example 1a) in CH₂Cl₂ (300 µl) was added at 25 °C TFA (452 mg, 306 µL, 3.97 mmol). The reaction mixture was stirred for 30 min. The reaction mixture was diluted with CH₂Cl₂ (15 mL) and cooled in an ice bath to 0 °C, adjusted with 10% Na₂CO₃-solution to pH∼10- and stirred for 10 min. The organic layer was separated and the aqueous layer was extracted with CH₂Cl₂ (3 x 20 mL) and CH₂Cl₂ : MeOH = 19:1 (2 x 20 mL). The combined organic layers were washed with aqueous saturated NaCl-solution, dried over Na₂SO₄, filtered and evaporated. to give a light yellow solid. The light yellow solid was subsequently purified two times by flash chromatography (silica gel, 4 g, 1 to 4% 2N NH₃/ MeOH in DCM) to yiled the title compound as a white solid (15.9 mg; 44 %). m/z = 455.2 [M+H]⁺, 1H NMR in CDCl3.

### Synthesis of the intermediate sulfinyl imines B4

### B4 (R² = F; R³ = Me): (R,E)-N-(1-(6-Bromo-3-fluoro-4-(triethylsilyl)pyridin-2-yl)ethylidene)-2-methylpropane-2-sulfinamide

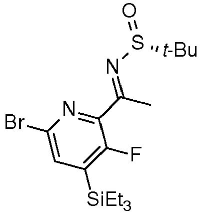

To a solution of 1-(6-bromo-3-fluoro-4-(triethylsilyl)pyridin-2-yl)ethanone, prepared according to Badiger, S. et al., int. patent application WO 2012095469A1, (8.13 g) in THF (59 ml) was added subsequently at 22 °C (R)-(+)-tert-butylsulfinamide (3.26 g) and titanium(IV)ethoxide (11.2 g) and the solution was stirred at 60 °C for 6 h. The mixture was cooled to 22 °C, treated with brine, the suspension was stirred for 10 min and filtered over dicalite. The layers were separated, the aqueous layer was extracted with ethyl acetate, the combined organic layers were washed with water, dried and evaporated. The residue was purified by flash chromatography (SiO₂, n-heptane/EtOAc, 5:1) to give the title compound (7.5 g, 70%) as a yellow oil. MS (ESI): m/z = 435.3, 437.3 [M+H]⁺.

### Synthesis of the intermediate pentafluoroketone hydrates B5

### B5 (R² = F; R³ = Me): (R)-N-((R)-2-(6-Bromo-3-fluoro-4-(triethylsilyl)pyridin-2-yl)-3,3,5,5,5-pentafluoro-4,4-dihydroxypentan-2-yl)-2-methylpropane-2-sulfinamide

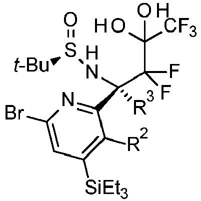

To a stirred solution of 1,1,1,3,3,3-hexafluoropropan-2-ol (26.9 g, 160 mmol, Eq: 1.3) in anhydrous tetrahydrofuran (247 ml) under argon atmosphere at -70 °C was slowly added n-butyllithium (1.6 M in hexane) (200 ml, 321 mmol, Eq: 2.6) keeping the internal temperature below -40 °C, then slowly allowing to reach 0 °C where stirring was continued for 1.5 h. The mixture was again cooled to -70 °C, a solution of (R,E)-N-(1-(6-bromo-3-fluoro-4-(triethylsilyl)pyridin-2-yl)ethylidene)-2-methylpropane-2-sulfinamide B4a (53.7 g, 123 mmol, Eq: 1) in tetrahydrofuran (61.7 ml) was added, and the mixture was then stirred at -70 °C to 23 °C for 16 h. Poured into brine, extracted with ethyl acetate, dried the organic layer over Na₂SO₄. Filtration and removal of the solvent in vacuum left a brown oil. The crude material was purified by flash chromatography (silica gel, 300 g, 0% to 50% EtOAc in heptane) to give the (R)-N-((R)-2-(6-bromo-3-fluoro-4-(triethylsilyl)pyridin-2-yl)-3,3,5,5,5-pentafluoro-4,4-dihydroxypentan-2-yl)-2-methylpropane-2-sulfinamide (56.3 g, 93.6 mmol, 75.9 % yield) as a light brown foam. MS (ESI): m/z = 583.1, 585.1 [M+H]⁺.

### Synthesis of the intermediate pentafluoroketone hydrates B6

### B6 (R² = F; R³ = Me): (R)-N-((R)-2-(6-Bromo-3-fluoropyridin-2-yl)-3,3,5,5,5-pentafluoro-4,4-dihydroxypentan-2-yl)-2-methylpropane-2-sulfinamide

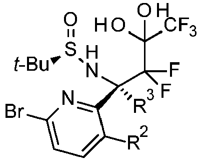

To a stirred solution of (R)-N-((R)-2-(6-bromo-3-fluoro-4-(triethylsilyl)pyridin-2-yl)-3,3,5,5,5-pentafluoro-4,4-dihydroxypentan-2-yl)-2-methylpropane-2-sulfinamide (56.3 g, 93.6 mmol, Eq: 1) in N,N-dimethylformamide (468 ml) and acetic acid (5.62 g, 5.36 ml, 93.6 mmol, Eq: 1) was added potassium fluoride (10.9 g, 187 mmol, Eq: 2) and the mixture was stirred at 23 °C for 16 h. Extracted with water an TBME, dried the organic layer over Na₂SO₄, filtered off and evaporated totally to give the crude product as a dark brown oil (60 g). The crude material was purified by flash chromatography (silica gel, 100 g, 0% to 40% EtOAc in heptane) to give: (R)-N-((R)-2-(6-bromo-3-fluoropyridin-2-yl)-3,3,5,5,5-pentafluoro-4,4-dihydroxypentan-2-yl)-2-methylpropane-2-sulfinamide (28.58 g, 58.7 mmol, 62.7 % yield) as a light brown foam. MS (ESI): m/z = 487.2, 489.2 [M+H]⁺.

### Synthesis of the intermediate pentafluoro alcohols B7

### B7a (R² = F; R³ = Me): (R)-N-((2R,4S)-2-(6-Bromo-3-fluoropyridin-2-yl)-3,3,5,5,5-pentafluoro-4-hydroxypentan-2-yl)-2-methylpropane-2-sulfinamide and B7b (R² = F; R³ = Me): (R)-N-((2R,4R)-2-(6-Bromo-3-fluoropyridin-2-yl)-3,3,5,5,5-pentafluoro-4-hydroxypentan-2-yl)-2-methylpropane-2-sulfinamide

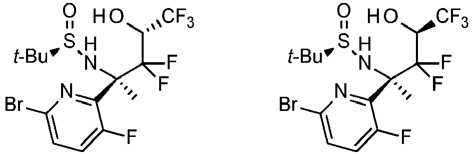

To a solution of (R)-N-((R)-2-(6-bromo-3-fluoropyridin-2-yl)-3,3,5,5,5-pentafluoro-4,4-dihydroxypentan-2-yl)-2-methylpropane-2-sulfinamide (25.28 g, 51.9 mmol, Eq: 1) in methanol (208 ml) was added portionwise at 0 °C sodium borohydride (3.93 g, 104 mmol, Eq: 2). After complete addition the reaction mixture was stirred at 23 °C for 1 h. poured onto ice water and diluted NH₄Cl-sol., then extracted twice with ethyl acetate. The organic layer was washed with brine, dried over Na₂SO₄, filtered off and evaporated totally. The residue was chromatographed (silica gel, 50 g, 0-50% EtOAc in heptane) to give (R)-N-((2R,4S)-2-(6-bromo-3-fluoropyridin-2-yl)-3,3,5,5,5-pentafluoro-4-hydroxypentan-2-yl)-2-methylpropane-2-sulfinamide (8.7 g, 18.5 mmol, 35.6 % yield) (faster eluting isomer: MS (ESI): m/z = 471.1, 473.1 [M+H]⁺) as an orange foam and (R)-N-((2R,4R)-2-(6-bromo-3-fluoropyridin-2-yl)-3,3,5,5,5-pentafluoro-4-hydroxypentan-2-yl)-2-methylpropane-2-sulfinamide (13.08 g, 27.8 mmol, 53.5 % yield) (slower eluting isomer: MS (ESI): m/z = 471.1, 473.1 [M+H]⁺) as an orange foam.

### Synthesis of the intermediate aminoalcohols B8

### B8a (R² = F; R³ = Me): (2S,4R)-4-Amino-4-(6-bromo-3-fluoropyridin-2-yl)-1,1,1,3,3-pentafluoropentan-2-ol

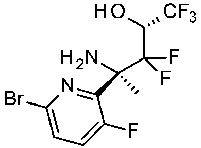

To a stirred solution of (R)-N-((2R,4S)-2-(6-bromo-3-fluoropyridin-2-yl)-3,3,5,5,5-pentafluoro-4-hydroxypentan-2-yl)-2-methylpropane-2-sulfinamide (9.83 g, 20.9 mmol, Eq: 1) was tetrahydrofuran (417 ml) at 23 °C was added conc. HCl (8.22 g, 6.85 ml, 83.4 mmol, Eq: 4) and the mixture was stirred for 1 h. Poured onto sat. NaHCO₃-sol. and extracted twice with EtOAc, dried over Na₂SO₄, filtered off and evaporated totally. The crude material was purified by flash chromatography (silica gel, 50 g, 0% to 30% EtOAc in heptane) to give the (2S,4R)-4-amino-4-(6-bromo-3-fluoropyridin-2-yl)-1,1,1,3,3-pentafluoropentan-2-ol (7.1 g, 19.3 mmol, 92.7 % yield) as a light brown solid. MS (ESI): m/z = 367.0, 369.0 [M+H]⁺.

### B8b (R² = F; R³ = Me): (2R,4R)-4-Amino-4-(6-bromo-3-fluoropyridin-2-yl)-1,1,1,3,3-pentafluoropentan-2-ol

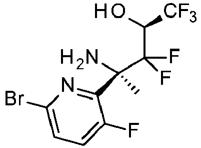

To a stirred solution of (R)-N-((2R,4R)-2-(6-bromo-3-fluoropyridin-2-yl)-3,3,5,5,5-pentafluoro-4-hydroxypentan-2-yl)-2-methylpropane-2-sulfinamide (5.23 g, 11.1 mmol, Eq: 1) in tetrahydrofuran (222 ml) at 23 °C was added conc. HCl (4.37 g, 3.65 ml, 44.4 mmol, Eq: 4) and stirred for 1 h. Poured onto sat. NaHCO₃-sol. and extracted twice with EtOAc, dried over Na₂SO₄, filtered off and evaporated totally. The crude material was purified by flash chromatography (silica gel, 50 g, 0% to 30% EtOAc in heptane) to give the (2R,4R)-4-amino-4-(6-bromo-3-fluoropyridin-2-yl)-1,1,1,3,3-pentafluoropentan-2-ol (2.41 g, 6.57 mmol, 59.2 % yield) as a light brown solid. MS (ESI): m/z = 367.0, 369.0 [M+H]⁺.

### Synthesis of the intermediate Boc-aminooxazines B10 (via intermediates B9)

### B10a (R² = F; R³ = Me): tert-butyl ((4R,6S)-4-(6-Bromo-3-fluoropyridin-2-yl)-5,5-difluoro-4-methyl-6-(trifluoromethyl)-5,6-dihydro-4H-1,3-oxazin-2-yl)carbamate

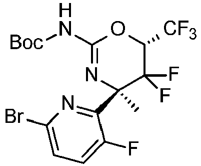

### Step 1: N-[[(1R,3S)-1-(6-Bromo-3-fluoro-2-pyridyl)-2,2,4,4,4-pentafluoro-3-hydroxy-1-methyl-butyl]carbamothioyl]benzamide

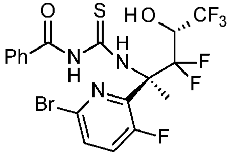

To a stirred solution of (2S,4R)-4-amino-4-(6-bromo-3-fluoropyridin-2-yl)-1,1,1,3,3-pentafluoropentan-2-ol (7.1 g, 19.3 mmol, Eq: 1) in tetrahydrofuran (446 ml) at 0 °C was added benzoyl isothiocyanate (3.16 g, 2.63 ml, 19.3 mmol, Eq: 1) and the mixtue was stirred at 23 °C for 1 h. All volatiles were removed in vacuum to give the crude thiourea which was directly used in the next step. MS (ESI): m/z = 530.1, 532.1 [M+H]⁺.

### Step 2 (intermediate B9a (R = Bz, R'= H; R² = F; R³ = Me): N-((4R,6S)-4-(6-Bromo-3-fluoropyridin-2-yl)-5,5-difluoro-4-methyl-6-(trifluoromethyl)-5,6-dihydro-4H-1,3-oxazin-2-yl)benzamide

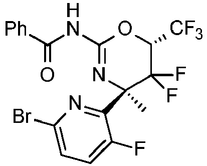

The above prepared crude thiourea was dissolved in acetonitrile (149 ml) at 23 °C, EDC·HCl (3.71 g, 19.3 mmol, Eq: 1) was added and this mixture was stirred at 80 °C for 2 h. Evaporated totally and the crude material was purified by flash chromatography (silica gel, 50 g, 0% to 40% EtOAc in heptane) to give the N-((4R,6S)-4-(6-bromo-3-fluoropyridin-2-yl)-5,5-difluoro-4-methyl-6-(trifluoromethyl)-5,6-dihydro-4H-1,3-oxazin-2-yl)benzamide (6.85 g, 13.8 mmol, 71.4 % yield) as a yellow solid. MS (ESI): m/z = 496.2, 498.2 [M+H]⁺.

### Step 3 (intermediate B9a (R = Bz, R'= Boc; R² = F; R³ = Me): tert-Butyl benzoyl((4R,6S)-4-(6-bromo-3-fluoropyridin-2-yl)-5,5-difluoro-4-methyl-6-(trifluoromethyl)-5,6-dihydro-4H-1,3-oxazin-2-yl)carbamate

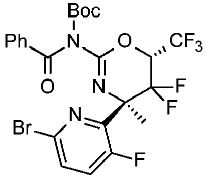

To a stirred solution of N-((4R,6S)-4-(6-bromo-3-fluoropyridin-2-yl)-5,5-difluoro-4-methyl-6-(trifluoromethyl)-5,6-dihydro-4H-1,3-oxazin-2-yl)benzamide (6.85 g, 13.8 mmol, Eq: 1) in tetrahydrofuran (368 ml) at 23 °C was added di-tert-butyl dicarbonate (Boc₂O) (3.31 g, 3.53 ml, 15.2 mmol, Eq: 1.1) and triethylamine (1.54 g, 2.12 ml, 15.2 mmol, Eq: 1.1) followed by DMAP (337 mg, 2.76 mmol, Eq: 0.2) and the mixture was stirred at 23 °C for 30 min. Removal of the solvent in vacuum at 23 °C left the crude doubly protected compound as a light yellow oil (8 g) which was used directly in the next step. MS (ESI): m/z = 596.2, 598.2 [M+H]⁺.

### Step 4: tert-butyl ((4R,6S)-4-(6-Bromo-3-fluoropyridin-2-yl)-5,5-difluoro-4-methyl-6-(trifluoromethyl)-5,6-dihydro-4H-1,3-oxazin-2-yl)carbamate

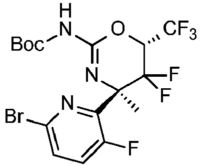

The above prepared crude doubly protected compound was dissolved in MeOH (552 ml), cooled to 0 °C, ammonia (7 M in MeOH) (197 ml, 1.38 mol, Eq: 100) was added and the mixture was stirred at 23 °C for 1 h. All volatiles were removed in vacuum and the crude material was purified by flash chromatography (silica gel, 100 g, 0% to 35% EtOAc in heptane) to give the tert-butyl ((4R,6S)-4-(6-bromo-3-fluoropyridin-2-yl)-5,5-difluoro-4-methyl-6-(trifluoromethyl)-5,6-dihydro-4H-1,3-oxazin-2-yl)carbamate (5.77 g, 11.7 mmol, 84.9 % yield) as a light yellow foam. MS (ESI): m/z = 492.2, 494.2 [M+H]⁺.

### BlOb (R² = F; R³ = Me): tert-butyl ((4R,6R)-4-(6-Bromo-3-fluoropyridin-2-yl)-5,5-difluoro-4-methyl-6-(trifluoromethyl)-5,6-dihydro-4H-1,3-oxazin-2-yl)carbamate

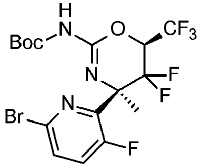

### Step 1: N-[[(1R,3R)-1-(6-Bromo-3-fluoro-2-pyridyl)-2,2,4,4,4-pentafluoro-3-hydroxy-1-methyl-butyl]carbamothioyl]benzamide

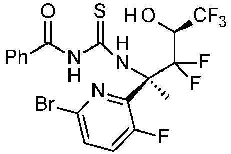

To a stirred solution of (2R,4R)-4-amino-4-(6-bromo-3-fluoropyridin-2-yl)-1,1,1,3,3-pentafluoropentan-2-ol (10.9 g, 29.7 mmol, Eq: 1) in tetrahydrofuran (986 ml) at 0 °C was added benzoyl isothiocyanate (4.85 g, 3.99 ml, 29.7 mmol, Eq: 1) and the mixtue was stirred at 23 °C for 1 h. All volatiles were removed in vacuum to give the crude thiourea which was directly used in the next step. MS (ESI): m/z = 530.1, 532.1 [M+H]⁺.

### Step 2 (intermediate B9b (R = Bz, R'= H; R² = F; R³ = Me): N-((4R,6R)-4-(6-Bromo-3-fluoropyridin-2-yl)-5,5-difluoro-4-methyl-6-(trifluoromethyl)-5,6-dihydro-4H-1,3-oxazin-2-yl)benzamide

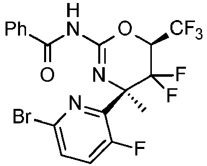

The above prepared crude thiourea was dissolved in acetonitrile (329 ml) at 23 °C, EDC·HCl (5.69 g, 29.7 mmol, Eq: 1) was added and this mixture was stirred at 80 °C for 2 h. Evaporated totally and the crude material was purified by flash chromatography (silica gel, 100 g, 0% to 40% EtOAc in heptane) to give the N-((4R,6R)-4-(6-bromo-3-fluoropyridin-2-yl)-5,5-difluoro-4-methyl-6-(trifluoromethyl)-5,6-dihydro-4H-1,3-oxazin-2-yl)benzamide (5.20 g, 10.5 mmol, 35.3 % yield) as a light yellow foam. MS (ESI): m/z = 496.0, 498.0 [M+H]⁺.

### Step 3 (intermediate B9b (R = Bz, R'= Boc; R² = F; R³ = Me): tert-Butyl benzoyl((4R,6R)-4-(6-bromo-3-fluoropyridin-2-yl)-5,5-difluoro-4-methyl-6-(trifluoromethyl)-5,6-dihydro-4H-1,3-oxazin-2-yl)carbamate

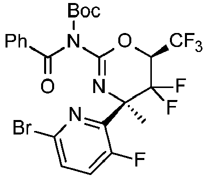

To a stirred solution of N-((4R,6R)-4-(6-bromo-3-fluoropyridin-2-yl)-5,5-difluoro-4-methyl-6-(trifluoromethyl)-5,6-dihydro-4H-1,3-oxazin-2-yl)benzamide (5.80 g, 11.7 mmol, Eq: 1) in tetrahydrofuran (312 ml) at 23 °C was added di-tert-butyl dicarbonate (Boc₂O) (2.81 g, 2.99 ml, 12.9 mmol, Eq: 1.1) and triethylamine (1.30 g, 1.79 ml, 12.9 mmol, Eq: 1.1) followed by DMAP (286 mg, 2.34 mmol, Eq: 0.2) and the mixture was stirred at 23 °C for 30 min. Removal of the solvent in vacuum at 23 °C left the crude doubly protected compound as a light yellow oil which was used directly in the next step. MS (ESI): m/z = 596.2, 598.2 [M+H]⁺.

### Step 4: tert-butyl ((4R,6R)-4-(6-Bromo-3-fluoropyridin-2-yl)-5,5-difluoro-4-methyl-6-(trifluoromethyl)-5,6-dihydro-4H-1,3-oxazin-2-yl)carbamate

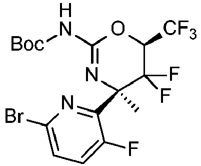

The above prepared crude doubly protected compound was dissolved in MeOH (468 ml), cooled to 0 °C, ammonia (7 M in MeOH) (167 ml, 1.17 mol, Eq: 100) was added and the mixture was stirred at 23 °C for 1 h. All volatiles were removed in vacuum and the crude material was purified by flash chromatography (silica gel, 100 g, 0% to 35% EtOAc in heptane) to give the tert-butyl ((4R,6R)-4-(6-bromo-3-fluoropyridin-2-yl)-5,5-difluoro-4-methyl-6-(trifluoromethyl)-5,6-dihydro-4H-1,3-oxazin-2-yl)carbamate (4.27 g, 8.68 mmol, 74.2 % yield) as an orange foam. MS (ESI): m/z = 492.2, 494.2 [M+H]⁺.

### Synthesis of the intermediate Boc-aminopyridine B11

### B11a (R² = F; R³ = Me): tert-Butyl ((4R,6S)-4-(6-amino-3-fluoropyridin-2-yl)-5,5-difluoro-4-methyl-6-(trifluoromethyl)-5,6-dihydro-4H-1,3-oxazin-2-yl)carbamate

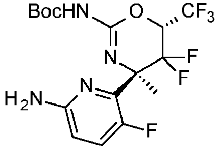

To a solution of tert-butyl ((4R,6S)-4-(6-bromo-3-fluoropyridin-2-yl)-5,5-difluoro-4-methyl-6-(trifluoromethyl)-5,6-dihydro-4H-1,3-oxazin-2-yl)carbamate B10a (1.05 g, 2.13 mmol, Eq: 1) in dioxane (40 ml) and water (13.3 ml) was added sodium azide (1.11 g, 17.1 mmol, Eq: 8), copper (I) iodide (163 mg, 853 µmol, Eq: 0.4), sodium ascorbate (84.5 mg, 427 µmol, Eq: 0.2) and (1R,2R)-N,N'-dimethyl-1,2-cyclohexanediamine (182 mg, 202 µl, 1.28 mmol, Eq: 0.6) and this mixture was stirred at 70 °C for 1 h. Poured onto sat. NaHCO₃-sol. and extracted twice with EtOAc, dried the combined organic layers over Na₂SO₄, filtered off and evaporated totally. The crude material was purified by flash chromatography (silica gel, 50 g, 0% to 50% EtOAc in heptane) to give title compound (84 mg, 196 µmοl, 9.19 % yield) as a colorless oil (MS (ESI): m/z = 429.3 [M+H]⁺) and the di-Boc material tert-butyl N-[(4R,6S)-4-(6-amino-3-fluoro-2-pyridyl)-5,5-difluoro-4-methyl-6-(trifluoromethyl)-6H-1,3-oxazin-2-yl]-N-tert-butoxycarbonyl-carbamate (480 mg, 908 µmοl, 42.6 % yield) as a light yellow foam which could also be used in the following step (MS (ESI): m/z = 529.4 [M+H]⁺).

### B11b (R² = F; R³ = Me): tert-Butyl ((4R,6R)-4-(6-amino-3-fluoropyridin-2-yl)-5,5-difluoro-4-methyl-6-(trifluoromethyl)-5,6-dihydro-4H-1,3-oxazin-2-yl)carbamate

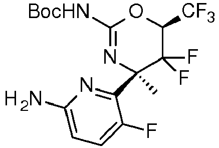

tert-Butyl ((4R,6R)-4-(6-bromo-3-fluoropyridin-2-yl)-5,5-difluoro-4-methyl-6-(trifluoromethyl)-5,6-dihydro-4H-1,3-oxazin-2-yl)carbamate B10b (2.05 g) was reacted with sodium azide in analogy to the preparation of compound B11a to give after flash chromatography (silica gel, 50 g, 0% to 50% EtOAc in heptane) the title compound (270 mg, 15%) as a yellow solid. MS (ESI): m/z = 429.3 [M+H]⁺. Also obtained di-Boc material tert-butyl N-[(4R,6R)-4-(6-amino-3-fluoro-2-pyridyl)-5,5-difluoro-4-methyl-6-(trifluoromethyl)-6H-1,3-oxazin-2-yl]-N-tert-butoxycarbonyl-carbamate (250 mg, 473 µmοl, 11.4 % yield) as a white foam which could also be used in the following step (MS (ESI): m/z = 529.4 [M+H]⁺).

### Synthesis of the intermediate Boc-amides B12 and deprotected amides I

### General procedure for the coupling of the Boc-aminopyridines B11 with the acid to the Boc-amide B12

Ghosez's reagent-method: To a suspension of the acid (197 µmol, Eq: 1.5) in dry dichloromethane (1.5 ml) at 0 °C was dropwise added 1-chloro-N,N,2-trimethylpropenylamine (Ghosez's reagent) (52.8 mg, 395 µmol, Eq: 3) and the mixture was stirred at 0 °C for 1 hour. This mixture was then added to a solution of the Boc-aminopyridine B11 (or corresponding di-Boc material) (132 µmol, Eq: 1.00) and diisopropylethylamine (51.0 mg, 69.0 µl, 395 µmοl, Eq: 3) in dry dichloromethane (1.5 ml) at 0 °C. The ice bath was removed and the mixture was stirred 1 to 16 hour(s) at ambient temperature. Evaporated totally at ambient temperature und directly purified by flash chromatography (silica gel, gradient of EtOAc in heptane) to give the Boc-amide B12 (or corresponding di-Boc material).

### General procedure for the deprotection of the Boc-amide B12 to the amide I

To a solution of the Boc-amide B12 (0.04 mmol) in dichloromethane (0.5 ml) was added at 22 °C trifluoroacetic acid (1.2 mmol) and stirring was continued for 1-16 h. The mixture was evaporated, the residue diluted with EtOAc and evaporated again. The residue was triturated with diethyl ether/pentane, the suspension was filtered and the residue dried to give the amide I. Alternative workup to obtain the free base: after stirring for 1-16 h, all volatiles were removed in vacuum, the residue was partitioned between EtOAc and sat. NaHCO₃-sol., the organic layer was washed with brine and dried over Na₂SO₄. Filtration and removal of the solvent in vacuum left the crude product which was purified by flash chromatography to give the amide I.

### B12a-1 (R² = F; R³ = Me): tert-Butyl ((4R,6S)-4-(6-(5-cyanopicolinamido)-3-fluoropyridin-2-yl)-5,5-difluoro-4-methyl-6-(trifluoromethyl)-5,6-dihydro-4H-1,3-oxazin-2-yl)carbamate

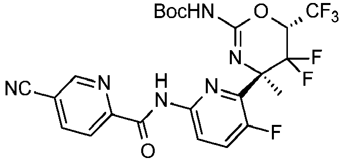

tert-Butyl ((4R,6S)-4-(6-amino-3-fluoropyridin-2-yl)-5,5-difluoro-4-methyl-6-(trifluoromethyl)-5,6-dihydro-4H-1,3-oxazin-2-yl)carbamate B11a (70 mg, 163 µmοl, Eq: 1) was coupled with 5-cyanopicolinic acid according to the Ghosez's reagent-method to give after flash chromatography (silica gel, 20 g, 0% to 70% EtOAc in heptane) the title compound (87 mg, 156 µmol, 95.3 % yield) as a white foam. MS (ESI): m/z = 559.3 [M+H]⁺.

### B12a-2 (R² = F; R³ = Me): tert-Butyl ((4R,6S)-4-(6-(5-cyano-3-methylpicolinamido)-3-fluoropyridin-2-yl)-5,5-difluoro-4-methyl-6-(trifluoromethyl)-5,6-dihydro-4H-1,3-oxazin-2-yl)carbamate

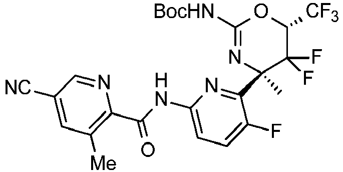

tert-Butyl ((4R,6S)-4-(6-amino-3-fluoropyridin-2-yl)-5,5-difluoro-4-methyl-6-(trifluoromethyl)-5,6-dihydro-4H-1,3-oxazin-2-yl)carbamate B11a (70 mg, 163 µmοl, Eq: 1) was coupled with 5-cyano-3-methylpicolinic acid according to the Ghosez's reagent-method to give after flash chromatography (silica gel, 20 g, 0% to 70% EtOAc in heptane) the title compound (88 mg, 154 µmοl, 94.1 % yield) as a white foam. MS (ESI): m/z = 573.4 [M+H]⁺.

### B12b-1 (R² = F; R³ = Me): tert-Butyl ((4R,6R)-4-(6-(5-cyanopicolinamido)-3-fluoropyridin-2-yl)-5,5-difluoro-4-methyl-6-(trifluoromethyl)-5,6-dihydro-4H-1,3-oxazin-2-yl)carbamate

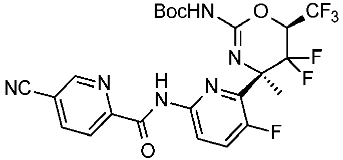

tert-Butyl ((4R,6R)-4-(6-amino-3-fluoropyridin-2-yl)-5,5-difluoro-4-methyl-6-(trifluoromethyl)-5,6-dihydro-4H-1,3-oxazin-2-yl)carbamate B11b (70 mg, 163 µmοl, Eq: 1) was coupled with 5-cyanopicolinic acid according to the Ghosez's reagent-method to give after flash chromatography (silica gel, 20 g, 0% to 70% EtOAc in heptane) the title compound (91 mg, 163 µmοl, 99.7 % yield) as a white foam. MS (ESI): m/z = 559.3 [M+H]⁺.

### B12b-2 (R² = F; R³ = Me): tert-Butyl ((4R,6R)-4-(6-(5-cyano-3-methylpicolinamido)-3-fluoropyridin-2-yl)-5,5-difluoro-4-methyl-6-(trifluoromethyl)-5,6-dihydro-4H-1,3-oxazin-2-yl)carbamate

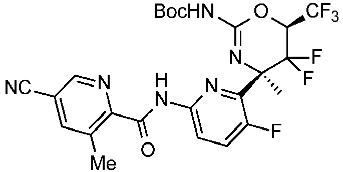

tert-Butyl ((4R,6R)-4-(6-amino-3-fluoropyridin-2-yl)-5,5-difluoro-4-methyl-6-(trifluoromethyl)-5,6-dihydro-4H-1,3-oxazin-2-yl)carbamate B11b (70 mg, 163 µmοl, Eq: 1) was coupled with 5-cyano-3-methylpicolinic acid according to the Ghosez's reagent-method to give after flash chromatography (silica gel, 20 g, 0% to 70% EtOAc in heptane) the title compound (60 mg, 105 µmol, 64.1 % yield) as a white solid. MS (ESI): m/z = 573.3 [M+H]⁺.

### Comparator 1

### N-(6-((4R,6S)-2-Amino-5,5-difluoro-4-methyl-6-(trifluoromethyl)-5,6-dihydro-4H-1,3-oxazin-4-yl)-5-fluoropyridin-2-yl)-5-cyanopicolinamide

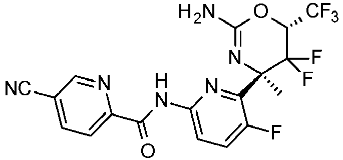

tert-butyl ((4R,6S)-4-(6-(5-cyanopicolinamido)-3-fluoropyridin-2-yl)-5,5-difluoro-4-methyl-6-(trifluoromethyl)-5,6-dihydro-4H-1,3-oxazin-2-yl)carbamate B12a-1 (80 mg, 143 µmol) was deprotected and the crude material was purified by flash chromatography (NH₂-silica gel, 10 g, 0% to 100% EtOAc in heptane) to give the title compound (52 mg, 113 µmοl, 79.2 % yield) as a white solid. MS (ESI): m/z = 459.3 [M+H]⁺.

### Comparator 2

### N-(6-((4R,6S)-2-Amino-5,5-difluoro-4-methyl-6-(trifluoromethyl)-5,6-dihydro-4H-1,3-oxazin-4-yl)-5-fluoropyridin-2-yl)-5-cyano-3-methylpicolinamide

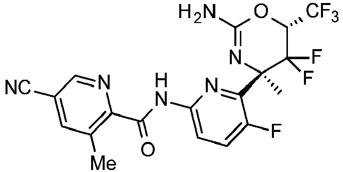

tert-Butyl ((4R,6S)-4-(6-(5-cyano-3-methylpicolinamido)-3-fluoropyridin-2-yl)-5,5-difluoro-4-methyl-6-(trifluoromethyl)-5,6-dihydro-4H-1,3-oxazin-2-yl)carbamate B12a-2 (88 mg, 154 µmol) was deprotected and the crude material was purified by flash chromatography (silica gel, 10 g, 0% to 60% EtOAc in heptane) to give the title compound (63 mg, 133 µmοl, 86.8 % yield) as a white solid. MS (ESI): m/z = 473.2 [M+H]⁺.

### Comparator 3

### N-(6-((4R,6R)-2-Amino-5,5-difluoro-4-methyl-6-(trifluoromethyl)-5,6-dihydro-4H-1,3-oxazin-4-yl)-5-fluoropyridin-2-yl)-5-cyanopicolinamide

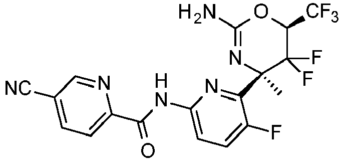

tert-butyl ((4R,6R)-4-(6-(5-cyanopicolinamido)-3-fluoropyridin-2-yl)-5,5-difluoro-4-methyl-6-(trifluoromethyl)-5,6-dihydro-4H-1,3-oxazin-2-yl)carbamate B12b-1 (85 mg, 152 µmol) was deprotected and the crude material was purified by flash chromatography (silica gel, 10 g, 0% to 60% EtOAc in heptane) to give the title compound (53 mg, 116 µmοl, 76 % yield) as a white solid. MS (ESI): m/z = 459.3 [M+H]⁺.

### Comparator 4

### N-(6-((4R,6R)-2-Amino-5,5-difluoro-4-methyl-6-(trifluoromethyl)-5,6-dihydro-4H-1,3-oxazin-4-yl)-5-fluoropyridin-2-yl)-5-cyano-3-methylpicolinamide

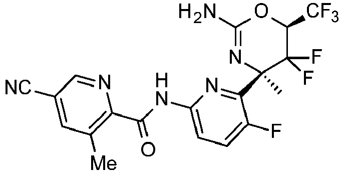

tert-Butyl ((4R,6R)-4-(6-(5-cyano-3-methylpicolinamido)-3-fluoropyridin-2-yl)-5,5-difluoro-4-methyl-6-(trifluoromethyl)-5,6-dihydro-4H-1,3 -oxazin-2-yl)carbamate B12b-2 (60 mg, 105 µmol) was deprotected and the crude material was purified by flash chromatography (silica gel, 10 g, 0% to 60% EtOAc in heptane) to give the title compound (49 mg, 104 µmοl, 99 % yield) as a white solid. MS (ESI): m/z = 473.3 [M+H]⁺.

## Claims

1. A compound of formula I, 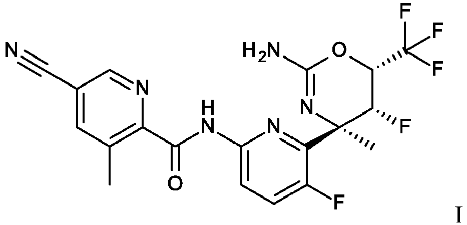 or pharmaceutically acceptable salts thereof.

2. A compound of formula I that is N-[6-[(4R,5R,6S)-2-amino-5-fluoro-4-methyl-6-(trifluoromethyl)-5,6-dihydro-1,3-oxazin-4-yl]-5-fluoro-2-pyridyl]-5-cyano-3-methylpyridine-2-carboxamide.

3. A process for preparing a compound of formula I as defined in any one of claims 1-2, which process comprises reacting a compound of formula XI' with a compound of formula XII' to a compound of formula I. 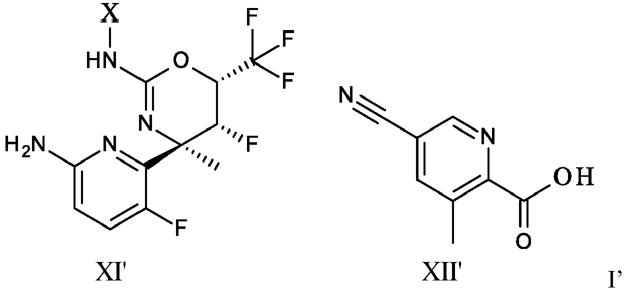 wherein X is an amino protecting group.

4. A compound of formula I according to any one of claims 1-2 for use as therapeutically active substance.

5. A compound of formula I according to any one of claims 1-2 for the use as therapeutically active substance for the therapeutic and/or prophylactic treatment of diseases and disorders **characterized by** elevated β-amyloid levels and/or β-amyloid oligomers and/or β-amyloid plaques or Alzheimer's disease.

6. A compound of formula I according to any one of claims 1-2 for the use as therapeutically active substance for the therapeutic and/or prophylactic treatment of amyotrophic lateral sclerosis (ALS), arterial thrombosis, autoimmune/inflammatory diseases, cancer such as breast cancer, cardiovascular diseases such as myocardial infarction and stroke, dermatomyositis, Down's Syndrome, gastrointestinal diseases, Glioblastoma multiforme, Graves Disease, Huntington's Disease, inclusion body myositis (IBM), inflammatory reactions, Kaposi Sarcoma, Kostmann Disease, lupus erythematosus, macrophagic myofasciitis, juvenile idiopathic arthritis, granulomatous arthritis, malignant melanoma, multiple mieloma, rheumatoid arthritis, Sjogren syndrome, SpinoCerebellar Ataxia 1, SpinoCerebellar Ataxia 7, Whipple's Disease or Wilson's Disease.

7. A pharmaceutical composition comprising a compound of formula I according to any one of claims 1-2 and a pharmaceutically acceptable carrier and/or a pharmaceutically acceptable auxiliary substance.

## Patentansprüche

1. Verbindung der Formel I 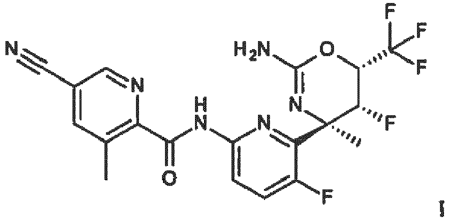 oder pharmazeutisch unbedenkliche Salze davon.

2. Verbindung der Formel I, die N-[6-[(4R,5R,6S)-2-Amino-5-fluor-4-methyl-6-(trifluormethyl)-5,6-dihydro-1,3-oxazin-4-yl]-5-fluor-2-pyridyl]-5-cyano-3-methyl-pyridin-2-carboxamid ist.

3. Verfahren zum Herstellen einer Verbindung der Formel I, wie in einem der Ansprüche 1-2 definiert, wobei das Verfahren das Reagieren einer Verbindung der Formel XI' mit einer Verbindung der Formel XII' zu einer Verbindung der Formel I umfasst 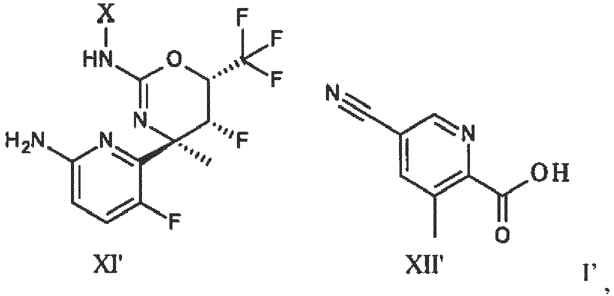 wobei X eine Aminoschutzgruppe ist.

4. Verbindung der Formel I nach einem der Ansprüche 1-2 zur Verwendung als therapeutisch wirksame Substanz.

5. Verbindung der Formel I nach einem der Ansprüche 1-2 zur Verwendung als therapeutisch wirksame Substanz für die therapeutische und/oder prophylaktische Behandlung von Krankheiten und Störungen, die durch erhöhte β-Amyloid-Spiegel und/oder β-Amyloid-Oligomere und/oder β-Amyloid-Plaques gekennzeichnet sind, oder Alzheimer-Krankheit.

6. Verbindung der Formel I nach einem der Ansprüche 1-2 zur Verwendung als therapeutisch wirksame Substanz für die therapeutische und/oder prophylaktische Behandlung von amyotropher Lateralsklerose (ALS), arterieller Thrombose, Autoimmun-/Entzündungskrankheiten, Krebs, wie Brustkrebs, kardiovaskulären Krankheiten, wie Myokardinfarkt und Schlaganfall, Dermatomyositis, Down-Syndrom, gastrointestinalen Krankheiten, Glioblastoma multiforme, Basedow-Krankheit, Huntington-Krankheit, Einschlusskörperchenmyositis (IBM), Entzündungsreaktionen, Kaposi-Sarkom, Kostmann-Krankheit, Lupus erythematodes, Makrophagenmyofasziitis, juveniler idiopathischer Arthritis, granulomatöser Arthritis, malignem Melanom, multiplem Myelom, Rheumatoidarthritis, Sjögren-Syndrom, spinozerebellärer Ataxie 1, spinozerebellärer Ataxie 7, Whipple-Krankheit oder Wilson-Krankheit.

7. Pharmazeutische Zusammensetzung, umfassend eine Verbindung der Formel I nach einem der Ansprüche 1-2 und einen pharmazeutisch unbedenklichen Träger und/oder eine pharmazeutisch unbedenkliche Hilfssubstanz.

## Revendications

1. Composé de formule I, 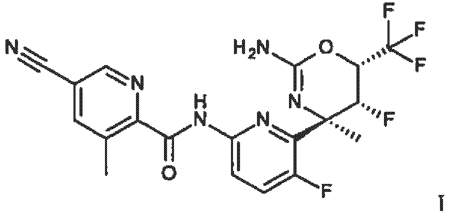 ou un de ses sels pharmaceutiquement acceptables.

2. Composé de formule I qui est le N-[6-[(4R,5R,6S)-2-amino-5-fluoro-4-méthyl-6-(trifluorométhyl)-5,6-dihydro-1,3-oxazin-4-yl]-5-fluoro-2-pyridyl]-5-cyano-3-méthyl-pyridine-2-carboxamide.

3. Procédé pour la préparation d'un composé de formule I tel que défini dans l'une quelconque des revendications 1 à 2, lequel procédé comprend la réaction d'un composé de formule XI' avec un composé de formule XII' pour donner un composé de formule I 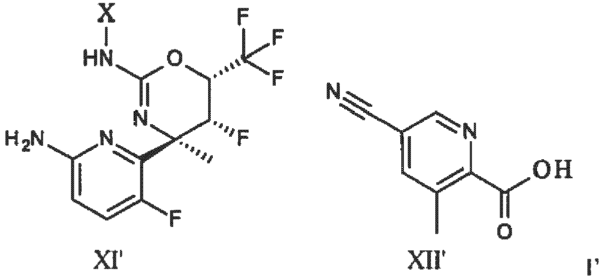 dans laquelle X représente un groupe amino-protecteur.

4. Composé de formule I selon l'une quelconque des revendications 1 à 2 pour une utilisation comme substance thérapeutiquement active.

5. Composé de formule I selon l'une quelconque des revendications 1 à 2 pour l'utilisation comme substance thérapeutiquement active pour le traitement thérapeutique et/ou prophylactique de maladies et de troubles **caractérisés par** des taux élevés de β-amyloïde et/ou d'oligomères β-amyloïdes et/ou de plaques β-amyloïdes ou de la maladie d'Alzheimer.

6. Composé de formule I selon l'une quelconque des revendications 1 à 2 pour l'utilisation comme substance thérapeutiquement active pour le traitement thérapeutique et/ou prophylactique de la sclérose latérale amyotrophique (SLA), de la thrombose artérielle, des maladies autoimmunes/inflammatoires, d'un cancer comme le cancer du sein, des maladies cardiovasculaires comme l'infarctus du myocarde et l'AVC, de la dermatomyosite, du syndrome de Down, des maladies gastro-intestinales, du glioblastome multiforme, de la maladie de Graves, de la maladie de Huntington, de la myosite à inclusions (IBM), des réactions inflammatoires, du sarcome de Kaposi, de la maladie de Kostmann, du lupus érythémateux, de la myofasciite à macrophages, de l'arthrite idiopathique juvénile, de l'arthrite granulomateuse, d'un mélanome malin, d'un myélome multiple, de la polyarthrite rhumatoïde, du syndrome de Sjögren, de l'ataxie spinocérébelleuse de type 1, de l'ataxie spinocérébelleuse de type 7, de la maladie de Whipple ou de la maladie de Wilson.

7. Composition pharmaceutique comprenant un composé de formule 1 selon l'une quelconque des revendications 1 à 2 et un véhicule pharmaceutiquement acceptable et/ou une substance auxiliaire pharmaceutiquement acceptable.
